(19) Europäisches Patentamt
European Patent Office
Office européen des brevets

(11) **EP 3 980 124 B1**

(12) **FASCICULE DE BREVET EUROPEEN**

(45) Date de publication et mention
de la délivrance du brevet:
**05.07.2023 Bulletin 2023/27**

(21) Numéro de dépôt: **21729247.3**

(22) Date de dépôt: **21.05.2021**

(51) Classification Internationale des Brevets (IPC):
*A61K 36/185* (2006.01)    *A61P 17/00* (2006.01)
*A23L 33/105* (2016.01)    *A61K 8/9789* (2017.01)
*A61Q 19/00* (2006.01)

(52) Classification Coopérative des Brevets (CPC):
(C-Sets disponibles)
**A61P 17/00; A23L 33/105; A23L 33/175;
A23L 33/18; A61K 8/645; A61K 8/9789;
A61K 36/185; A61Q 19/00;** A23V 2002/00;
A61K 2236/00; A61Q 19/007; A61Q 19/02;
A61Q 19/06; A61Q 19/08                (Cont.)

(86) Numéro de dépôt international:
**PCT/EP2021/063707**

(87) Numéro de publication internationale:
**WO 2021/234166 (25.11.2021 Gazette 2021/47)**

(54) **EXTRAIT DU TOURTEAU DES GRAINES DE MORINGA PEREGRINA, SON PROCÉDÉ D'OBTENTION ET SON UTILISATION DANS DES COMPOSITIONS COSMÉTIQUES OU NUTRICOSMÉTIQUES**

EXTRAKT AUS MORINGA-PEREGRINA-SAMENKUCHEN, VERFAHREN ZU SEINER HERSTELLUNG UND SEINE VERWENDUNG IN KOSMETISCHEN ODER NUTRIKOSMETISCHEN ZUSAMMENSETZUNGEN

EXTRACT OF MORINGA PEREGRINA SEED CAKE, METHOD FOR OBTAINING SAME AND USE THEREOF IN COSMETIC OR NUTRICOSMETIC COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.05.2020 FR 2005433
16.03.2021 FR 2102631**

(43) Date de publication de la demande:
**13.04.2022 Bulletin 2022/15**

(73) Titulaire: **Agence Francaise pour le Développement d'Alula
75008 Paris (FR)**

(72) Inventeurs:
• **DODINET, Elizabeth
12560 Saint-Laurent-d'Olt (FR)**
• **BOURGETEAU, Vincent
56130 Férel (FR)**

(74) Mandataire: **Loyer & Abello
9, rue Anatole de la Forge
75017 Paris (FR)**

(56) Documents cités:
**CN-A- 107 012 190    FR-A1- 2 776 519**

• **H M ABU-TARBOUSH ET AL: "Characterization of Hydrolysates Produced by Enzymatic hydrolysis of Camel Casein and Protein Isolates of Al-Ban (Moringa peregrina) and Karkade (Hibiscus sabderiffa) Seeds", J. SAUDI SOCFOR AGRIC. SCI., vol. 4, no. 2, 1 janvier 2005 (2005-01-01) , pages 61-82, XP055753092,**

- NASHEF A S ET AL: "EFFECTS OF ALKALI ON PROTEINS. DISULFIDES AND THEIR PRODUCTS", JOURNAL OF AGRICULTURAL AND FOOD CHEMISTRY, AMERICAN CHEMICAL SOCIETY, BOOKS AND JOURNALS DIVISION|, vol. 25, no. 2, 1 mars 1977 (1977-03-01), pages 245-251, XP001087830, ISSN: 0021-8561, DOI: 10.1021/JF60210A020
- RAO M B ET AL: "Molecular and biotechnological aspects of microbial proteases", MICROBIOLOGY AND MOLECULAR BIOLOGY REVIEWS, AMERICAN SOCIETY FOR MICROBIOLOGY, US, vol. 62, no. 3, 1 septembre 1998 (1998-09-01), pages 597-635, XP002245348, ISSN: 1092-2172
- T. Shantha Raju: "Proteolysis of Proteins" In: "Co? and Post-Translational Modifications of Therapeutic Antibodies and Proteins", 2 avril 2019 (2019-04-02), John Wiley & Sons, Inc, Hoboken, NJ, USA, XP055753173, ISBN: 978-1-119-05331-6 pages 183-202, DOI: 10.1002/9781119053354.ch15, page 184
- YOUNG-SHICK HONG ET AL: "Molecular Weight Distribution of Protein Hydrolysate by the Enzymic Hydrolysis of Weakly Acid-Treated Wheat Gluten", FOOD SCIENCE AND TECHNOLOGY RESEARCH, vol. 7, no. 2, 1 janvier 2001 (2001-01-01), pages 126-130, XP055753367, CH ISSN: 1344-6606, DOI: 10.3136/fstr.7.126
- ZHOU XU ET AL: "Purification and identification immunomodulatory peptide from rice protein hydrolysates", FOOD AND AGRICULTURAL IMMUNOLOGY., vol. 30, no. 1, 1 janvier 2019 (2019-01-01), pages 150-162, XP055753384, GB ISSN: 0954-0105, DOI: 10.1080/09540105.2018.1553938
- MENDEL FRIEDMAN ET AL: "Protein-Alkali Reactions: Chemistry, Toxicology, and Nutritional Consequences", RETINAL DEGENERATIVE DISEASES, SPRINGER, US, PAGE(S) 367 - 412 , 1 janvier 1984 (1984-01-01), XP009524239, ISBN: 978-1-4684-4790-3 Extrait de l'Internet: URL:https://link.springer.com/chapter/10.1007/978-1-4684-4790-3_18
- KJAER A ET AL: "ISOTHIOCYANATES IN MYROSINASE-TREATED SEED EXTRACTS OF MORINGA PEREGRINA", PHYTOCHEMISTRY, ELSEVIER, AMSTERDAM, NL, vol. 18, no. 9, 1 janvier 1979 (1979-01-01), pages 1485-1487, XP009024211, ISSN: 0031-9422, DOI: 10.1016/S0031-9422(00)98480-2 cité dans la demande
- AFSHARYPUORA S ET AL: "Volatile constituents of the seed kernel and leaf of Moringa peregrina (forssk.) Fiori, Agricolt. cultivated in Chabahar (IRAN)", IRANIAN JOURNAL OF PHARMACEUTICAL SCIENCES, IRANIAN SOCIETY OF PHARMACEUTICAL SCIENTISTS, IR, vol. 6, no. 2, 1 janvier 2010 (2010-01-01) , pages 141-144, XP009508062, ISSN: 1735-2444 cité dans la demande
- AL-DABBAS MAHER M ET AL: "Chemical composition and oil components in seeds of Moringa peregrina (Forssk) Fiori", CROP RESEARCH, AGRICULTURAL RESEARCH INFORMATION CENTRE, HISAR, IN, vol. 40, 1 janvier 2010 (2010-01-01), pages 161-167, XP009163458, ISSN: 0970-4884
- A. P. De Groot ET AL: "Effects of Severe Alkali Treatment of Proteins on Amino Acid Composition and Nutritive Value", The Journal of Nutrition, vol. 98, no. 1, 1 May 1969 (1969-05-01), pages 45-56, XP055753138, US ISSN: 0022-3166, DOI: 10.1093/jn/98.1.45

(52) Classification Coopérative des Brevets (CPC): (Cont.)

C-Sets
A23V 2002/00, A23V 2200/318

## Description

### Domaine technique

L'invention se rapporte au domaine de la cosmétique et nutricosmétique et plus particulièrement celui des ingrédients actifs entrant dans la formulation des compositions pour les soins de la peau. L'invention a trait à un extrait du tourteau des graines de *Moringa peregrina* comprenant majoritairement un hydrolysat peptidique. L'invention concerne également le procédé d'obtention de l'extrait particulier du tourteau des graines de *Moringa peregrina,* les compositions cosmétiques ou nutricosmétiques comprenant de tels extraits, et enfin l'utilisation cosmétique ou nutricosmétique de telles compositions pour le soin de la peau, du cuir chevelu et des phanères.

### Arrière-plan technologique

Les Moringaceae constituent une famille mono-générique (un seul genre, *Moringa* Adans), élément de la flore Saharo-sindienne, constituée d'entre douze et quatorze espèces selon les auteurs, réparties de l'Afrique orientale à l'Asie. Le genre est classiquement divisé en 3 sections qui ne sont, cependant, pas confirmées comme monophylétiques par les analyses phylogénétiques. Ces dernières ont plutôt mis en évidence des clades axés sur certains caractères morphologiques : pachycaules (« bottle trees ») ; tubéreux (« tuberous trees ») et ni pachycaules - ni tubéreux (en anglais « slender trees »). L'espèce *Moringa peregrina* (Forssk.) Fiori, appartient au troisième groupe. Les quelques études génétiques sur le genre ou la famille confirment la réalité de l'espèce par rapport aux autres espèces dans le genre, notamment vis-à-vis du Moringa indien, *Moringa oleifera* Lam. (voir notamment les articles: OLSON, M. E. 2002, Combining Data from DNA Sequences and Morphology for a Phylogeny of Moringaceae (Brassicales), Systematic Botany 27(1): 55-73; HASSANEIN, A. M. A. ET AL-SOQEE, A. A., 2018, Morphological and genetic diversity of Moringa oleifera and Moringa peregrina génotypes, Horticulture, Environment and Biotechnology 59(2): 251-261). Un article récent sur le *Moringa peregrina* échantillonné sur différentes localités en Arabie Saoudite a conclu, en utilisant des marqueurs ITS, à une stabilité génétique de l'espèce (ALAKLABI, A., 2015, Genetic diversity of Moringa peregrina species in Saudi Arabia with ITS sequences, Saudi Journal of Biological Sciences 22: 186-190) avec cependant un haut niveau de variation génétique intra-populations.

L'espèce *Moringa peregrina* se retrouve dans les environnements rocheux du Yémen, d'Oman, et d'Arabie Saoudite, en Afrique orientale, au Soudan, en Éthiopie, Érythrée, Somalie et Djibouti. Sa présence en Iran semble limitée aux provinces sud-orientales mais demande à être confirmée (PROTA14 = MUNYANZIZA E. ET YONGABI K. A., Vegetable oils/Oléagineux, *Moringa peregrina* (Forssk.) Fiori, http://database.prota.org/protahtml/moringa peregrina_fr.htm, accès le 23/10/2019). Au Levant et en Égypte, l'espèce ne représente, aujourd'hui, plus que de rares stations dispersées, relictuelles (à l'exception de quelques peuplements altitudinaux), principalement dans les secteurs de l'aire soudanienne. *Moringa peregrina* est également considéré aujourd'hui comme rare et en danger au Soudan et au Yémen. *Moringa peregrina* occupe par rapport aux autres espèces de son clade les habitats les plus arides et les plus inhospitaliers. Il est apparemment plus résistant à la sécheresse que *Moringa oleifera* qui est planté commercialement à grande échelle dans les zones tropicales et subtropicales. Des travaux récents ont montré que la taille et la grosseur des graines impactait favorablement le temps de germination et le taux et la rapidité de la croissance des jeunes individus (GOMAA N. H. ET PICO F. X., 2011, Seed germination, seedling traits, and seed bank of the tree Moringa peregrina (Moringaceae) in a hyper-arid environment, American Journal of Botany 98(6): 1024-1030), indiquant un ajustement dans l'allocation des ressources sur la qualité de la graine plutôt que le nombre, ce qui permet à *Moringa peregrina* de se reproduire efficacement dans des environnements abiotiques extrêmes (hyperarides). Les graines de *Moringa peregrina* ont une mesotesta centrale plus épaisse, en termes de couche de cellules, que celles de *Moringa oleifera.*

Il existe quelques mentions historiques qui tendent à indiquer que l'huile de *Moringa peregrina* faisait l'objet d'un commerce actif dans les débuts de l'Islam dans la région d'AlUla (NASEEF, A. A. S.,1995, *Al-'Ulā, A study of Cultural and Social Heritage).* L'huile produite à partir de *Moringa peregrina* est aujourd'hui destinée principalement à l'autoconsommation ou aux marchés locaux. En Arabie Saoudite, les feuilles étaient utilisées traditionnellement en décoction en usage interne pour soigner le diabète, les maladies du colon, les maladies des yeux et les anémies (ABDEL-KADER, M. S., HAZAZI A. M. A., ELMAKKI O. A. ET ALQASOUMI S. I., 2018, A survey on the traditional plants used in Al Kobah village, Saudi Pharmaceutical Journal 26(6): 817-821) et comme diurétique, rubéfiant et astringent (AQEEL A. A. M. , TARIQ M., MOSSA J. S., AL-YAHYA M. A. ET AL-SAID M. S., 1984, « Plants used in Arabia Folk medicine » , Report submitted to Saudi Arabian National Cenre for Science and Technology, Riyadh, Saudi Arabia). En Oman, l'huile, extraite par les femmes à la fin de l'été, est utilisée contre les migraines, la fièvre, les brûlures, les lacérations et les fractures, la constipation et les douleurs d'estomac, contre les douleurs musculaires et contre la sécheresse capillaire (GHAZANFAR S. A., 1994, Handbook of Arabian Medicinal Plants, 1e éd., CRC Press, Boca Raton, Ann Harbor, s.u. ; GHAZANFAR S.A., 1998, Plants of Economic Importance, cap. 15, in GHAZANFAR, S.A. ET FISHER, M. (éd.) Végétation of the Arabian Peninsula. Geobotany 25, p. 241-264, Kluwer Academic Publishers, tableau 11.1, p. 247 et 11.7 p. 251). Elle était également utilisée dans des compositions parfumées (GHAZANFAR S. A., 1998, p. 259) et en Oman et au Yémen comme lotion faciale (GHAZANFAR S. A. ET RECHINGER B., 1996, Two multi-purpose seed oils form Oman. Plants for Food and Medicine. Paper presented at the joint meeting of the Society for Economic Botany and International Society for Ethnopharmacology, 1-7 July 1996, London).

On connaît des extraits provenant de la graine de *Moringa oleifera,* dans le domaine cosmétique. Par exemple on connaît du document FR296879 un extrait de graines entières (avec téguments) de *Moringa oleifera* contenant pour 100 g d'extrait sec de 5 à 50% d'huile (dont triglycérides, acides gras et lipides polaires) et de 0.01 à 5% de polyphénols, et son utilisation dans des compositions cosmétiques pour lutter contre le vieillissement cutané. Dans ce document, il est effectué une extraction par un solvant moyennement polaire sur la graine entière de *Moringa oleifera.*

On connaît également du document FR2776519 que des extraits protéiques de graines de *Moringa oleifera,* connus pour leurs effets clarifiants sur les eaux turbides, présentent sur la peau et les muqueuses un effet adoucissant, conditionneur physiologique, hydratant, restructurant, réparateur et un effet antipollution. Dans ce document les principes actifs sont des protéines de poids moléculaires compris entre 6500 et 8800 Da qui sont obtenues par extraction aqueuse sur le tourteau de *Moringa oleifera* avec de l'hydroxyde de sodium 4N pendant une heure à pH 7,5.

On connaît encore du document FR2825267 que des extraits de graines de *Moringa,* entières ou broyées, décortiquées ou non décortiquées, ou dont on a réalisé un broyat ou des extraits protéiniques délipidés ou non délipidés, ont des bénéfices dans les domaines de la désodorisation et de la suppression des mauvaises odeurs, de la propreté, de l'hygiène intime, de l'hygiène buccale et des soins dentaires, de même que des propriétés cosmétiques adoucissantes, hydratantes, apaisantes et anti-fatigue sur la peau, Dans ce document, il est démontré que les extraits de protéines entières présentent une activité accrue lorsque la graine n'est pas déshuilée.

Le document FR3076460 concerne l'utilisation d'un extrait protéique de graine non germée et déshuilée de *Moringa oleifera* pour le traitement des peaux et/ou muqueuses sensibles, sensibilisées, réactives, fragiles et/ou fragilisées et/ou dans le traitement et/ou la prévention de l'érythème, en particulier l'érythème fessier du nourrisson. Dans ce document, le procédé d'extraction permet d'obtenir une fraction majeure de protéines dont les poids moléculaires sont environ de 8 800 Da.

Enfin, on connaît du document KR20130088224 l'utilisation d'un extrait de graine entière germée de *Moringa oleifera* en cosmétique, en particulier obtenu par une extraction à l'aide d'un fluide supercritique. Ce procédé permet d'isoler des caroténoïdes et acides aminés apolaires, qui sont décrits comme actifs pour une utilisation cosmétique blanchissante.

Tous les documents précités concernent l'utilisation de l'espèce *Moringa oleifera* ; aucun ne décrit l'utilisation dans le domaine cosmétique d'extraits provenant de l'espèce *Moringa peregrina.*

Plus spécifiquement pour l'espèce *Moringa peregrina,* on connait que certains composés phénoliques et flavonoïdes obtenus de feuilles ou de la graine entière de *Moringa peregrina* présentent une activité anti-oxydante (AL-DABBAS M., 2017, Antioxidant activity of différent extracts from the aerial part of Moringa peregrina (Forssk.) Fiori, from Jordan, Pakistan Journal of Pharmaceutical Sciences, 30(6): 2151-2157). Ces composés sont extraits avec des solvants tels que le méthanol, l'éthyl acétate ou l'hexane à partir des feuilles ou des graines entières. Il apparaît que ce sont les feuilles qui comprennent le plus de composés actifs. On connait également le document de ABU TARBOUSH *et al.* XP055753092, 2005 qui décrit l'extraction d'un hydrolysat à partir de graines de *Moringa peregrina* décortiquées produit par hydrolyse enzymatique pendant une durée de 10 heures. Le but de cette extraction est d'obtenir un produit présentant une haute capacité d'absorption de l'huile et de l'eau. Enfin, le document de NASHEF *et al.,* XP0010830, 1977, décrit de manière générale les conséquences de l'hydrolyse alcaline principalement sur les protéines pures pouvant entrainer des modifications des propriétés physiques et chimiques des protéines ainsi que de leur valeur nutritive. Les conditions d'hydrolyse utilisées avec 0,1 M d'hydroxyde de sodium sont à une température de 50°C pendant une durée de 24 heures. Le document invoque des lésions rénales chez le rat nourri avec des protéines traitées aux alcalis dans ces conditions.

Ainsi, selon l'espèce utilisée dans le genre *Moringa,* il est observé que selon la partie des plantes (feuille, graine), la partie des graines (entière ou non, décortiquée ou non) et le procédé d'extraction mis en oeuvre, notamment le choix du solvant, les molécules extraites s'avèrent être différentes. Or la composition de l'extrait conditionne l'activité biologique et en conséquence l'efficacité cosmétique.

Considérant ce qui précède, un problème que se propose de résoudre l'invention est de développer des produits nouveaux à base d'un extrait de l'espèce *Moringa peregrina* du genre *Moringa* et de la famille Moringaceae qui soient utilisables en cosmétique et facile à mettre en oeuvre.

De ce fait, la Demanderesse a mis en évidence un nouvel extrait obtenu à partir du tourteau des graines de l'espèce *Moringa peregrina,* qui améliore l'aspect de la peau, des muqueuses et des phanères et notamment pour prévenir et/ou lutter contre la sécheresse de la peau et des muqueuses, prévenir et/ou à lutter contre les signes cutanés du vieillissement et/ou du photo-vieillissement, favoriser le blanchiment de la peau et prévenir les tâches de vieillesse, ainsi que pour favoriser l'amincissement et prévenir ou soulager les rougeurs de la peau. L'extrait selon l'invention comprend majoritairement un hydrolysat peptidique du tourteau des graines de *Moringa peregrina*. L'extrait obtenu spécifiquement à partir du tourteau des graines non décortiquées de *Moringa peregrina* est nouveau à double titre dans le domaine de la cosmétique par rapport aux extraits de l'art antérieur, d'une part par l'espèce spécifique d'origine utilisée et d'autre part par son profil moléculaire particulier.

Par accord intergouvernemental du 10 avril 2018 entre le gouvernement de la République française et le Royaume d'Arabie Saoudite, la demanderesse, Agence Française Pour Le Développement d'AlUla (AFALULA) ainsi que la Commission Royale pour AlUIA (RCU) ont pour projet commun notamment de développer une agriculture responsable et l'économie locale, notamment par la production locale de produits naturels dérivés des plantes indigènes et de protéger la biodiversité et les droits de la région d'AlUla du Royaume d'Arabie Saoudite. Le Royaume d'Arabie Saoudite est membre du Protocole de Nagoya depuis le 8 octobre 2020. Au moment de la rédaction du présent brevet, le règlement d'exécution en vertu duquel le Protocole de Nagoya sera intégré dans les aspects pertinents du droit local est en cours d'examen. Par conséquent, à ce stade, le Royaume d'Arabie Saoudite n'a pas d'exigences spécifiques en ce qui concerne cette demande de brevet et le Protocole de Nagoya. Ainsi, au jour du dépôt de la demande de brevet, il n'y a aucune exigence de certificat de conformité concernant l'accès aux ressources génétiques.

## Résumé

L'invention a pour premier objet un extrait du tourteau des graines de *Moringa peregrina* comprenant majoritairement un hydrolysat peptidique qui comprend des dérivés d'acides aminés, des acides aminés, des peptides et glycopeptides dont le poids moléculaire est compris entre 100 Da et 6 000 Da, préférentiellement entre 1 500 Da et 5 000 Da, préférentiellement encore entre 3 000 et 4 500 Da, et en ce qu'il est obtenu des graines non décortiquées et à maturité du fruit de *Moringa peregrina* par protéolyse chimique à pH supérieur à 13 pendant une durée d'environ 2 heures à une température comprise entre 16 et 25°C.

Grâce à sa caractéristique, l'extrait selon l'invention est unique dans le genre *Moringa* et la famille Moringaceae. Il sera démontré que l'extrait de l'espèce *Moringa peregrina* présente un hydrolysat peptidique particulier et différent de ceux connus des autres espèces du genre, notamment de l'espèce *Moringa oleifera,* que la demanderesse a su mettre en évidence.

L'invention a pour deuxième objet un procédé d'obtention d'un extrait de tourteau des graines de *Moringa peregrina* selon l'invention, caractérisé en ce qu'il comprend les étapes suivantes selon lesquelles :

a) on recueille les graines non décortiquées et récoltées à maturité du fruit de *Moringa peregrina* que l'on sèche pour obtenir un taux d'humidité interne inférieur à 8%,
b) on presse les graines séchées de sorte à séparer l'huile du reste de la graine de sorte à obtenir le tourteau comprenant moins de 6% en poids d'huile résiduelle,
c) on broie le tourteau obtenu à l'étape b),
d) on met en dispersion en phase aqueuse le tourteau broyé obtenu à l'étape c),
e) on réalise une protéolyse chimique de la dispersion aqueuse obtenue à l'étape d) pendant une durée d'environ 2 heures, à pH supérieur à 13 et à température comprise entre 16 et 25°C,
f) on neutralise la protéolyse pour stabiliser l'hydrolysat peptidique obtenu,
g) on récupère l'hydrolysat peptidique par séparation solide/liquide,
h) on purifie l'hydrolysat peptidique par ultrafiltration, puis éventuellement,
i) on effectue une lyophilisation de l'hydrolysat peptidique obtenu à l'étape h).

L'invention a pour troisième objet une composition cosmétique ou nutricosmétique comprenant à titre d'agent actif, une quantité efficace d'un extrait du tourteau des graines de *Moringa peregrina* selon l'invention et un excipient physiologiquement acceptable.

L'invention a enfin pour quatrième objet l'utilisation cosmétique ou nutricosmétique d'une composition selon l'invention, pour améliorer l'aspect de la peau, des muqueuses ou des phanères, prévenir et/ou lutter contre la sécheresse de la peau et des muqueuses, prévenir et/ou à lutter contre les signes cutanés du vieillissement et/ou du photo-vieillissement, favoriser le blanchiment de la peau et favoriser l'amincissement.

## Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.

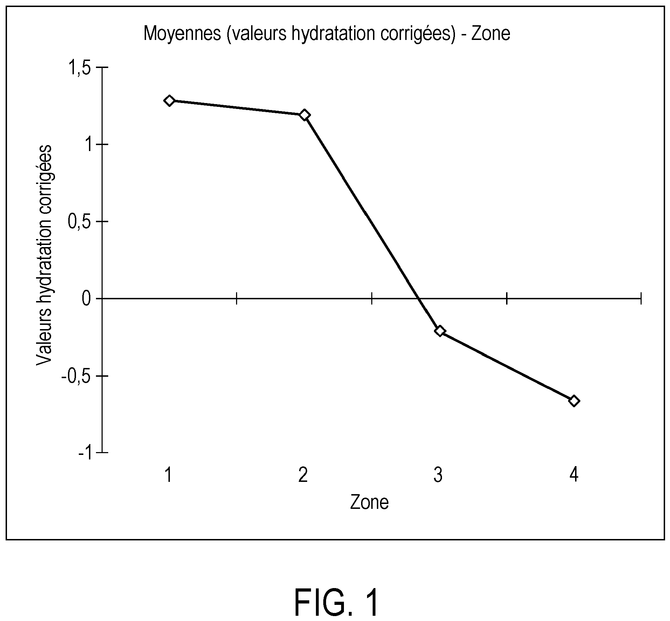

[fig.1] représente les résultats comparatifs de l'effet hydratant de différents produits à la surface de la peau.

**Description des modes de réalisation**

Dans cette description, à moins qu'il n'en soit spécifié autrement, il est entendu que, lorsqu'un intervalle est donné, il inclut les bornes supérieures et inférieures dudit intervalle.

Dans la présente invention, les abréviations suivantes signifient :

- MTT : bromure de 3-(4,5-dimethylthiazol-2-yl)-2,5-diphényl tétrazolium (le test MTT est une méthode rapide pour la numération des cellules vivantes)

- SDS : Sodium Dodecyl Sulfate

- PBS : Phosphate Buffer Saline

- ELISA : Enzyme-Linked Immunosorbent Assay

- PCR : Polymerase Chain Reaction

- ANOVA : Analysis Of Variance

- MSH : Mélanocyte Stimulating Hormone

Dans la présente invention, on entend par :

- « majoritairement un hydrolysat peptidique » une quantité supérieure à 10%, préférentiellement supérieure à 20%, préférentiellement encore supérieure à 30% et pouvant atteindre environ 40% (Masse/Masse) de matière sèche en peptides, oligopeptides, glycopeptides et en acides aminés ou leurs dérivés nitrilés volatiles, préférentiellement encore 50% du poids de la matière sèche.

- « quantité efficace » la quantité nécessaire de molécules actives pour obtenir le résultat recherché, à savoir, permettre notamment d'améliorer l'aspect de la peau.

- « protéolyse » la segmentation des protéines en peptides, oligopeptides et ses fragments de base (acides aminés) via l'hydrolyse chimique ou enzymatique.

- « application topique », le fait d'appliquer ou d'étaler le principe actif selon l'invention, ou une composition le contenant, à la surface de la peau, d'une muqueuse ou des phanères.

- « physiologiquement acceptable » qui convient à une utilisation topique, en contact avec la peau humaine, ou à une utilisation par d'autres voies d'administration, par exemple la voie orale ou l'injection dans la peau, sans risque de toxicité, d'incompatibilité, d'instabilité, de réponse allergique.

- « tourteau », la partie déshuilée de la graine après pressage. Il s'agit du résidu solide de l'extraction de l'huile des graines. C'est un coproduit de la trituration, procédé de fabrication de l'huile. Il représente généralement de 50 à 75% de la masse des graines.

- « graines non décortiquées », le fait que la coque (péricarpe) et le tégument des graines récoltées soient maintenus autour des amandes.

- « à maturité du fruit », le fait que le fruit soit mûr, préférentiellement lorsque la gousse est en début de déhiscence et qu'elle prend une coloration beige foncé à brune et lorsqu'une torsion exercée de 180° sur le quart inférieur de la gousse provoque l'amorce de l'ouverture des valves.

- « environ » une marge plus ou moins de 10% à 20% par rapport à l'information donnée.

  - « pool de molécules actives », également « principe actif », l'hydrolysat peptidique extrait selon le procédé de l'invention à partir du tourteau des graines de *Moringa peregrina.* Cet hydrolysat est responsable des activités biologiques décrites dans la présente invention.

- « agent actif » une quantité suffisante d'un extrait selon l'invention pour obtenir les activités biologiques décrites. Selon si l'extrait est liquide ou séché, concentré ou non, les quantités de l'agent actif peuvent varier dans les proportions de 0,002 à 40% en poids par rapport au poids total de la composition.

- « signes du vieillissement cutané », toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, toute dégradation interne de la peau consécutive à une exposition aux rayonnements ultraviolets (UV).

L'invention concerne en premier objet un extrait du tourteau des graines de *Moringa peregrina* comprenant majoritairement un hydrolysat peptidique qui comprend des dérivés d'acides aminés, des acides aminés, des peptides et glycopeptides dont le poids moléculaire est compris entre 100 Da et 6 000 Da, préférentiellement entre 1 500 Da et 5 000 Da, préférentiellement encore entre 3 000 et 4 500 Da, et en ce qu'il est obtenu des graines non décortiquées et à maturité du fruit de *Moringa peregrina* par protéolyse chimique à pH supérieur à 13 pendant une durée d'environ 2 heures à une température comprise entre 16 et 25°C.

L'hydrolysat peptidique, comprenant des peptides, oligopeptides, glycopeptides et acides aminés, tel que dans le profil défini ci-dessous, n'a jamais été mis en évidence dans un extrait des graines du genre *Moringa peregrina.* L'espèce *Moringa peregrina* pousse dans des climats très arides. Ainsi, sa faculté à s'adapter à la sécheresse et à se reproduire dans des conditions extrémophiles lui a permis d'acquérir des caractéristiques particulières uniques, que la Demanderesse a pu identifier par la mise en oeuvre d'un procédé d'extraction spécifique sur le tourteau des graines de *Moringa peregrina.*

Dans le cadre de la présente invention, la partie de plante choisie est le tourteau des graines de *Moringa peregrina.* Il est connu que les graines de *Moringa peregrina* sont utilisées pour l'extraction de leur huile, utile pour l'autoconsommation ou en soins médicinaux traditionnels divers. Le tourteau obtenu après que la graine a été déshuilée, est un déchet actuellement utilisé pour la nourriture des animaux notamment.

Selon un mode de réalisation préférentiel, l'extrait selon l'invention est obtenu du tourteau des graines non décortiquées de *Moringa peregrina.*

Selon un mode de réalisation, l'hydrolysat peptidique de l'extrait selon l'invention comprend des dérivés d'acides aminés, des acides aminés, des peptides et glycopeptides dont le poids moléculaire est compris entre 100 Da et 6000 Da, et plus particulièrement entre 1 500 Da et 5 000 Da, et encore plus particulièrement entre 3 000 Da et 4 500 Da.

Selon un autre mode de réalisation, l'extrait comprend également entre 0.3 % et 3 % de composés volatiles, dont 50% de ces composés, soit entre 0,15% et 1,5% de l'extrait selon l'invention, est constitué de composés nitrilés légers, avec principalement l'isobutyronitrile et le méthyl-butanénitrile ; dont 5 à 10% de ces composés soit entre 0,015 et 0,3% de l'extrait selon l'invention est constitué de dérivés d'isothiocyanates, avec principalement l'isothiocyanate d'isopropyle et l'isothiocyanate d'isobutyle ; dont 1 à 5% de ces composés soit entre 0,003 et 0,15 % de l'extrait selon l'invention est constitué d'huile essentielle, avec principalement de l'Eucalyptol, du Menthol et du Benzaldéhyde.

L'invention a pour deuxième objet un procédé d'un extrait de tourteau des graines de *Moringa peregrina* selon l'invention comprenant les étapes suivantes selon lesquelles :

a) on recueille les graines non décortiquées et récoltées à maturité du fruit de *Moringa peregrina* que l'on sèche pour obtenir un taux d'humidité interne inférieur à 8%,
b) on presse les graines séchées de sorte à séparer l'huile du reste de la graine de sorte à obtenir le tourteau comprenant moins de 6% en poids d'huile résiduelle,
c) on broie le tourteau obtenu à l'étape b),
d) on met en dispersion en phase aqueuse le tourteau broyé obtenu à l'étape c),
e) on réalise une protéolyse chimique de la dispersion aqueuse obtenue à l'étape d) pendant une durée d'environ 2 heures, à pH supérieur à 13 et à température comprise entre 16 et 25°C,
f) on neutralise la protéolyse pour stabiliser l'hydrolysat peptidique obtenu,
g) on récupère l'hydrolysat peptidique par séparation solide/liquide,
h) on purifie l'hydrolysat peptidique par ultrafiltration, puis éventuellement,
i) on effectue une lyophilisation de l'hydrolysat peptidique obtenu à l'étape h).

On recueille les graines non décortiquées, c'est à dire dont on garde la coque (péricarpe), à maturité du fruit et préférentiellement lorsque la gousse est en début de déhiscence.

On sèche les graines pour obtenir un taux d'humidité interne inférieur à 8% et préférentiellement autour de 6% ; le séchage se fera préférentiellement sur clayette ventilée à l'abri des rayons du soleil de préférence sous ombrière à l'air libre.

On broie alors les graines séchées extemporanément au pressage à froid qui permet de séparer mécaniquement l'huile du reste de la graine compressée à savoir le tourteau.

Le tourteau est alors broyé mécaniquement avec tout type de broyeurs mécaniques tels que système à marteaux, à fléau, à couteaux, à concassage/déchiquetage, à billes ou boulets ou à pilon, mais aussi avec tout type de cryobroyeur.

La dispersion en phase aqueuse selon l'étape d) et la protéolyse selon l'étape e) s'effectuent avantageusement toujours sous agitation permettant ainsi une dispersion et une homogénéisation du solide dans le liquide, améliorant ainsi la surface d'échange globale et par conséquent la protéolyse.

On obtient un hydrolysat peptidique liquide d'une densité supérieure à 1 et préférentiellement autour de 1.1, comprenant un taux de matières sèches compris entre 10 et 15 %, préférentiellement autour de 12.5% comprenant entre 1 et 6% de composés azotés, notamment des dérivés nitriles volatiles dans une proportion de 0.5 à 1.5%, préférentiellement autour de 0.8% et 20 mg/litre de polyphénols (0.0002%).

Selon un mode de réalisation préférentiel du procédé selon l'invention, la séparation solide/liquide de l'étape g) est réalisée par différents procédés tels que centrifugation, essorage, filtration.

Selon un autre mode de réalisation préférentiel du procédé selon l'invention, l'ultrafiltration à l'étape h) est effectuée avec un seuil de coupure compris entre 100 Da et 6 000 Da et plus particulièrement entre 1 500 Da et 5 000 Da, et encore plus particulièrement entre 3 000 Da et 4 500 Da.

Dans un mode de réalisation selon l'invention, le procédé d'obtention d'un extrait de tourteau des graines est obtenu à partir du tourteau des graines non décortiquées par protéolyse, sous agitation, dans une proportion au plus de 25% en poids de matière solide par rapport au poids total mis en oeuvre dans le solvant aqueux, à une température comprise entre 16 et 25°C pendant une durée d'environ 2 heures.

Dans un mode de réalisation du procédé d'obtention de l'extrait de *Moringa peregrina* liquide obtenu, l'hydrolysat peptidique est purifié par distillation, microfiltration, ultrafiltration et/ou nanofiltration pour concentrer l'extrait en composés d'intérêt par rapport aux dérivés nitriles volatiles également extraits, Ces étapes de purification permettent de concentrer le pool de composés d'intérêt au dépend d'autres composés extraits tels que cités.

Dans un autre mode de réalisation du procédé d'extraction selon l'invention, l'extrait liquide obtenu est séché de manière à obtenir un extrait sec du tourteau des graines de *Moringa peregrina* contenant une quantité supérieure à 10%, préférentiellement supérieure à 20%, préférentiellement encore supérieure à 30% et pouvant atteindre jusqu'à environ 40% (Masse/Masse) de matière sèche en peptides, oligopeptides, glycopeptides et en acides aminés ou leurs dérivés nitrilés volatiles, préférentiellement encore 50% du poids de la matière sèche.

Selon un mode de réalisation de l'invention l'extrait de tourteau des graines de *Moringa peregrina* liquide obtenu est préférentiellement séché par exemple par atomisation, lyophilisation ou zéodratation de manière à obtenir un extrait de graines de *Moringa peregrina* solide, l'eau étant évaporée. Le séchage peut être réalisé en présence d'un additif de support de séchage tel que la maltodextrine, la cyclodextrine ou l'inuline, ou en présence d'un support inorganique tel que phyllosilicate, magnésium silicate ou carbonate et ses sels.

L'invention concerne également l'extrait du tourteau des graines de *Moringa peregrina* susceptible d'être obtenu par le procédé d'extraction selon l'invention.

L'invention a pour troisième objet une composition cosmétique ou nutricosmétique comprenant à titre d'agent actif, une quantité efficace d'un extrait du tourteau des graines de *Moringa peregrina* selon l'invention, et un excipient physiologiquement acceptable.

La composition selon l'invention peut être formulée sous la forme de différentes préparations adaptées à une administration topique ou à une administration orale.

Selon une première variante, les différentes préparations sont adaptées à l'administration topique et incluent les crèmes, les émulsions huile dans eau et eau dans huile, les laits, les pommades, les lotions, les huiles, les baumes, les solutions aqueuses ou hydro-alcooliques ou glycoliques, les sérums, les poudres, les patchs, les sprays ou tout autre produit pour application externe tel que par, exemple, les dispositifs médicaux ou les produits de la cosméto-textile.

Selon une deuxième variante, les différentes préparations sont adaptées à une administration orale ; l'extrait végétal comprenant l'hydrolysat peptidique pouvant entrer soit dans une composition alimentaire soit dans un complément alimentaire. Le complément alimentaire peut se présenter sous forme de gélules ou de capsules molles de gélatine ou végétales dans le cadre de la présente invention. Ledit complément alimentaire peut alors contenir de 0.01 à 100% en poids de l'extrait végétal.

Dans le cadre d'une utilisation alimentaire, à visée nutritive ou cosmétique (cosmeto-food ou nutricosmétique), la composition sera avantageusement formulée sous la forme d'une préparation adaptée à une administration orale. Elle pourra ne pas comprendre d'excipient et être constituée, en intégralité, de l'extrait végétal comprenant l'hydrolysat peptidique sous forme séchée.

Selon un mode de réalisation préférentiel, les compositions selon l'invention sont destinées plus particulièrement à une administration par voie topique. Ces compositions doivent donc contenir un milieu cosmétiquement acceptable, c'est-à-dire compatible avec la peau et les phanères, et couvrent toutes les formes cosmétiques. Ces compositions pourront notamment être sous forme de crèmes, émulsions huile-dans-eau, ou eau-dans-huile ou émulsions multiples, sérums, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, émollients, épaississants, diluants, tensioactifs, anti-oxydants, agents bioactifs, colorants, conservateurs, parfums. Elles peuvent être utilisées comme produit de soin et/ou comme produit de maquillage de la peau.

La composition selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non d'un rinçage, ou encore sous forme de shampooing. La composition selon l'invention pourra avantageusement être utilisée dans les soins antipelliculaires. Elle peut également se présenter sous forme de teinture ou de mascara à appliquer au pinceau ou au peigne, en particulier sur les cils, les sourcils ou les cheveux.

Les compositions selon l'invention comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

L'INCI Dictionary & Handbook (« International Nomenclature of Cosmetic Ingrédients » (13e éd. 2010) publié par The Personal Care Products Council Inc., Washington, D.C.) décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

Dans tous les cas, l'homme de métier veillera à ce que ces adjuvants ainsi que leurs proportions soient choisis de telle manière à ne pas nuire aux propriétés avantageuses recherchées de la composition selon l'invention.

Selon un mode de réalisation avantageux de l'invention, la quantité ou teneur en extrait végétal dans la composition cosmétique selon l'invention est de 0.002 à 20 % en poids, et en particulier de 0.01 à 10 % en poids par rapport au poids total de la composition.

Selon un autre mode de réalisation avantageux de l'invention, la quantité ou teneur en extrait végétal dans la composition nutricosmétique selon l'invention est de 0.01 à 100% en poids par rapport au poids total de la composition. Plus préférentiellement, la quantité en extrait végétal est de 0.02 à 40 % en poids, et en particulier de 0.2 à 20 % en poids par rapport au poids total de la composition.

L'invention a pour quatrième objet l'utilisation cosmétique ou nutricosmétique d'une composition selon l'invention pour améliorer l'aspect de la peau, des muqueuses ou des phanères, prévenir et/ou lutter contre la sécheresse de la peau et des muqueuses, prévenir et/ou à lutter contre les signes cutanés du vieillissement et/ou du photo-vieillissement, favoriser le blanchiment de la peau et pour favoriser l'amincissement.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

L'usage du verbe « comporter », « comprendre » ou « inclure » et de ses formes conjuguées n'exclut pas la présence d'autres éléments ou d'autres étapes que ceux énoncés dans une revendication.

**Exemples**

Exemple 1 : Préparation d'un extrait végétal selon l'invention à partir du tourteau de *Moringa peregrina*

Des graines de *Moringa peregrina* (Forssk.) Fiori obtenues à maturité du fruit ont été séchées pour obtenir un taux d'humidité interne inférieur à 8% et préférentiellement autour de 6%, puis pressées avec une presse mécanique à vis sans fin, de sorte à séparer l'huile du reste de la graine pour obtenir d'une part l'huile vierge peregrina (nom INCI « Moringa peregrina seed oil ») et d'autre part un tourteau. Le tourteau est alors isolé sous forme de boudins prédécoupés en morceau de 1 à 2 cm sur lequel est effectué l'extraction. La composition des matières premières utilisées dans le procédé est donnée ci-dessous.

[Tableau 1]

| Matières | Fournisseur | % | Qté réelle pesée |
|---|---|---|---|
| Tourteau de *peregrina* *prédécoupé en morceaux de 1 à 2 cm | | 8.4% | 33.6 |
| Eau du réseau | | 80.68% | 322.7 |

(suite)

| Matières | Fournisseur | % | Qté réelle pesée |
|---|---|---|---|
| Hydroxyde de sodium | PROCHIMIA | 3.36 % | 13.5 |
| Acide citrique monohydrate F6000 | ADETIS | 7.14% | 28.7 |
| Benzoate de sodium | ADETIS | 0.42% | 1.7 |
| | Total | 100 | 400.2 |

Protocole :

a) On prépare une solution concentrée à 1 molaire d'un agent alcalin fort tel que notamment l'hydroxyde de sodium, l'hydroxyde de potassium ou l'hydroxyde de calcium, ou d'un mélange aqueux concentrant 1 molaire d'agents alcalins forts, mais préférentiellement l'hydroxyde de sodium ; cette solution alcaline forte présente un pH supérieur à 13,

b) On pèse dans un ratio [1 : 10] (masse/masse) du tourteau de *peregrina* prédécoupé en morceaux de 1cm environ dans de la solution alcaline,

c) on broie le tourteau obtenu à l'étape b),

d) On met en dispersion en phase aqueuse le tourteau broyé obtenu à l'étape c),

e) on réalise une protéolyse chimique de la dispersion aqueuse obtenue à l'étape d) pendant une durée d'environ 2 heures à une température de 20°C,

f) on neutralise la protéolyse pour stabiliser l'hydrolysat peptidique obtenu avec un acide faible de sorte à tamponner la solution dans une plage de pH 4.5-5.5,

g) on récupère l'hydrolysat peptidique par séparation solide/liquide,

h) on purifie l'hydrolysat peptidique par ultrafiltration.

On obtient un filtrat jaune translucide contenant environ 12,48% de matière sèche. L'extrait liquide obtenu,est nommé par la suite « hydrolysat peptidique *peregrina* selon l'invention » ou « hydrolysat peptidique *peregrina* ». Cet extrait liquide est utilisé par la suite pour les tests d'efficacité.

Cet hydrolysat peptidique *peregrina* selon l'invention présente une densité supérieure à 1 et préférentiellement autour de 1,1, comprenant un taux de matières sèches compris entre 10 et 15 %, préférentiellement autour de 12,5% comprenant entre 1 et 6% de composés azotés, principalement des peptides et glycopeptides et également des dérivés nitriles volatiles dans une proportion de 0,5 à 1,5%, préférentiellement autour de 0,8% et 20 mg/litre de polyphénols (0.0002%). La composition des composés volatils de l'hydrolysat *peregrina* selon l'invention se trouve ci-après.

[Tableau 2]

| Tr | N° CAS | Composés | % MS |
|---|---|---|---|
| 4.42. | 64-17-5 | Ethanol | 0.809 |
| 5.58 | 67-64-1 | Acétone | 0.444 |
| 6.10 | 75-15-0 | Disulfure de carbone | 0.327 |
| 7.83 | 78-93-3 | 2-Butanone | 0.165 |
| 8.57 | 141-78-6 | Acétate d'Ethyle | 0.047 |
| 8.99 | 78-82-0 | Isobutyronitrile | 20.720 |
| 10.39 | 78-82-0 | Isobutyronitrile | 0.127 |

(suite)

| Tr | N° CAS | Composés | % MS |
|---|---|---|---|
| 12.06 | 547-63-7 | Isobutyrate de méthyle | 0.201 |
| 14.10 | 18936-17-9 | 2-Méthyl-butanenitrile | 2.194 |
| 14.56 | 625-28-5 | 3-Méthyl-butanenitrile | 27.495 |
| 18.77 | 66-25-1 | Hexanal | 0.186 |
| 21.09 | 2253-73-8 | Isothiocyanate d'isopropyle | 4.590 |
| 23.65 | 628-73-9 | Hexanenitrile | 0.198 |
| 24.43 | 110-43-0 | 2-Heptanone | 0.093 |
| 27.18 | 4426-79-3 | 2-isothiocyanato-Butane | 1.058 |
| 27.59 | 80-56-8 | Alpha-Pinène | 0.048 |
| 28.45 | 591-82-2 | Isothiocyanate d'isobutyle | 2.299 |
| 28.99 | 100-52-7 | Benzaldéhyde | 1.753 |
| 29.77 | 108-95-2 | Phénol | 0.413 |
| 30.80 | 13475-82-6 | 2,2,4,6,6-pentaméthyl-Heptane | 0.318 |
| 32.80 | 99-87-6 | Para-Cymène | 0.232 |
| 33.10 | 138-86-3 | Limonène | 0.133 |
| 33.34 | 470-82-6 | Eucalyptol | 0.918 |
| 36.45 | 1195-32-0 | Para-Cyménène | 0.035 |
| 36.88 | 124-19-6 | Nonanal | 0.080 |
| 40.26 | 65-85-0 | Acide benzoïque | 19.150 |
| 41.07 | 1490-04-6 | Menthol | 0.918 |
| 56.29 | 96-76-4 | 2,4-Di-telt-butylphénol | 0.086 |
| | | Total | 85.035 |

L'hydrolysat peptidique peregrina contient de l'isothiocyanate d'isopropyle et de l'isothiocyanate d'isobutyle à des niveaux relativement élevés, confirmant les publications antérieures sur l'espèce (KJAER et al. 1979, Isothiocyanates in Myrosinase-treated seed extracts of Moringa peregrina, Phytochemistry, 18, p. 1485-1487 ; AFSHARYPUOR et al., 2010, Volatile Constituents of the Seed Kernel and Leaf of Moringa peregrina (Forssk.) Fiori, Agricolt. Cultivated in Chabahar (Iran), Iranian Journal of Pharmaceutical Sciences 6(2): 141-144 ; DEHSHAHRI S. et al., 2012, Détermination of volatile glucosinate dégradation products in seed coat, stem and in vitro cultures of Moringa peregrina (Forssk.) Fiori, ScienceOpen, Research in Pharmaceutical Sciences 7(1) : 51-56). Les isothiocyanates sont des composés produits par diverses plantes appartenant à l'ordre des Brassicales, notamment dans les familles des Brassicaceae, Capparaceae, Caricaceae et Moringaceae comme système de défense contre les attaques de pathogènes. Dans le genre *Moringa,* ils ont été identifiés notamment dans M. *oleifera* et *M. stenopetala* (Baker f.) Cufold. (ABD RANI N.Z., KHARAINA, H. et KuMOLOSAS), E., 2018, Moringa genus: A review of Phytochemistry and Pharmacology, Frontiers in Pharmacology, vol. 9, art. 108). Les isothiocyanates proviennent de l'hydrolyse des glucosinolates par l'enzyme myrosinase lorsque les tissus de la plante sont endommagés. Il a été rapporté que les isothiocyanates ont divers effets biologiques, tels qu'une activité antifongique (TRONCOSO-ROJAS, R. ET TIZNADO-HERNANDEZ, M. E., 2007, Natural compounds to control fungal diseases in fruits & vegetables, in TRONCOSO-ROJAS, R., TIZNADO-HERNANDEZ, M. E., GONZALEZ-LEON, A. (éd) Recent advances in alternative postharvest technologies to control fungal diseases in fruits & vegetables. Editorial. Transworld Research Network, Kerala, India, pp 127-156 ; TRONCOSO-ROJAS, R. et al., 2005, Analysis of the isothiocyanates présent in cabbage leaves extract and their potential application to control Alternaria rot in bell peppers, Food Research International 38:701-708), des effets anti-microbiens, anti-cancéreux et anti-inflammatoires (PARK, E.J. et al., 2011, Inhibition of lipopolysaccharide induced cyclooxygenase-2 expression and inducible nitric oxyde synthase by 4-[(2-O-acetyl-$\alpha$-L-rhamnosyloxy)benzyl]isothiocyanate from M. oleifera, Nutrition and Cancer 63(6): 971-982 ; RAJAN, T. S. et al., 2016, Anticancer activity of glucomoringin isothiocyanate in human malignant astrocytoma cells, Fitoterapa 110 :1-7 ; Padla E. P. et al., 2012, Antimicrobial isothiocyanates from the seeds of Moringa oleifera Lam., Zeitschrift für Naturforschung C, 67, 557-564 ; WATERMAN, C. et al., 2014, Stable, water extractable isothiocyanates from Moringa oleifera leaves attenuate inflammation in vitro. Phytochemistry 103: 114-122). Le furfural est présent à une concentration très standard, trouvée dans ce type de graines et dans de nombreux fruits secs. Le disulfure de carbone, l'isobutyronitrile, l'isobutyrate de méthyle, le méthyl butanenitrile et l'hexanenitrile sont des composés volatils issus des acides aminés. Ce sont des marqueurs de la dégradation des protéines. Ces composés de dégradation représentent environ 50% des composés volatils de l'extrait du tourteau de *peregrina* hydrolysé. Ce résultat indique que l'extrait est principalement constitué de protéines ou peptides. Il n'a été détecté que de très faibles traces de graisses ou de sucres libres dans l'extrait hydrolysé de *Moringa peregrina,* et d'huiles essentielles, celles-ci représentant 4% des composés volatils avec la présence de Menthol à environ 1%, la présence d'Eucalyptol à 1% et la présence de Benzaldhéyde à environ 2%. Dans la matière sèche, le tampon citrate (qui est un composé osidique) représente environ 50% du reste et englobe les composés glycosylés (osidiques) résultant de la dégradation par protéolyse des protéines, oligopeptides et acides aminés. Enfin, il est à noter la présence d'environ 20 mg/litre de polyphénols (0,0002%) issus des graines de *peregrina.* L'acide benzoïque n'est pas à considérer dans la caractérisation car il s'agit d'un agent stabilisant ajouté à l'extrait. Ce filtrat liquide compose l'extrait selon l'invention et est utilisé pur dans les tests qui suivent.

La matière sèche de l'extrait décrite ci-dessus est obtenue par une méthode gravimétrique basée sur la masse avant et après évaporation présent dans l'extrait liquide. Cette masse sèche se compose principalement de protéines et glycoprotéines, dont le poids moléculaire est compris entre 100 et 6000 Da, partiellement dégradées et hydrosolubles que l'on appelle des oligopeptides ou des glyco-oligopeptides ou hydrolysat peptidique selon l'invention, issus de l'hydrolyse du tourteau de *Moringa peregrina.* L'extrait sec comprend aussi un tampon citrate de sodium qui stabilise ces oligopeptides et glyco-oligopeptides dans un état hydrolysé statique. Enfin cet extrait est microbiologiquement stabilisé avec un conservateur hydrosoluble comme le benzoate de sodium.

Exemple 2 : Effet de l'hydrolysat peptidique *peregrina* selon l'invention comme antioxydant

L'objectif de cette étude est d'évaluer la modulation de l'activité anti-oxydante par l'hydrolysat peptidique *peregrina* dans un modèle colorimétrique *in vitro* acellulaire faisant appel au radical DPPH (2,2-diphenyl-1-picrylhydrazyl) ainsi qu'à l'antioxydant de référence, l'acide ascorbique. La méthode utilisée est dite d'inhibition. Elle est basée sur la dégradation du radical oxydant DPPH, de coloration violette absorbant à 540 nm, par un antioxydant de référence, l'acide ascorbique. Cette réaction sert de témoin positif et conduit à la formation du composé DPPH qui est incolore voire jaune clair. L'hydrolysat peptidique *peregrina* selon l'invention et le produit de référence « Acide Ascorbique » sont mis en contact avec la solution de DPPH pendant 30 mn à 40°C. L'activité antioxydante est alors évaluée par mesure d'absorbance à 540 nm. La modulation de cette activité est exprimée en pourcentage de stimulation de l'activité antioxydante par l'actif testé, avec pour référence l'activité antioxydante maximale obtenue en présence d'acide ascorbique $(T^+)$.

Protocole : Une solution de DPPH est incubée, pendant 30 minutes à 40°C, en absence (contrôle), en présence de l'hydrolysat peptidique de *peregrina* selon l'invention $(T^+)$ et à des concentrations décroissantes de l'échantillon à tester. A la fin de la période d'incubation, l'activité antioxydante en présence du produit de référence et en présence ou en absence de l'hydrolysat peptidique *peregrina* a été révélée par coloration après 30 minutes à 40°C. Elle a ainsi été évaluée par mesure d'absorbance du milieu réactionnel à 540 nm. Pour chaque concentration testée, la modulation de l'activité antioxydante par le produit testé est calculée selon la formule suivante.

$$\text{[Math. 1] Pourcentage de modulation de l'activité antioxydante} = 100 \times [(DO_{540} \text{ Contrôle} - DO_{540} \text{ Produit à tester}) / DO_{540} \text{ Produit de référence}].$$

Si le résultat est négatif, le produit à tester sera considéré comme oxydant ; si le résultat est positif, le pourcentage sera exprimé en stimulation de l'activité anti-radicalaire. Les résultats obtenus sont donnés ci-après de l'inhibition du DPPH par l'hydrolysat peptidique *peregrina* selon l'invention.

[Tableau 3]

| | | Inhibition DPPH |
|---|---|---|
| Hydrolysat peptidique *peregrina* selon l'invention | 2% | 12 |
| | 1.0% | 13 |
| | 0.1% | 2 |

Conclusion : L'hydrolysat peptidique *peregrina* selon l'invention est capable de protéger des radicaux libres, il présente des propriétés anti-oxydantes significatives dès la concentration de 1%.

Exemple 3 : Effet de l'extrait *peregrina* selon l'invention comme inhibiteur des Métalloprotéases

L'objectif de cette étude est d'évaluer la modulation de l'activité anti-Métalloprotéase par l'hydrolysat peptidique *peregrina* selon l'invention dans un modèle *in vitro* acellulaire faisant appel à une Collagénase de type I et une Hyaluronidase, un complexe de substrat et un chromophore, Ninhydrine. Une solution tamponnée de Collagénase de type I et Hyaluronidase réagit avec un complexe de substrat spécifique et le transforme pour former un composé capable d'activer un chromophore par incubation à 80°C pendant 15 mn. L'activité de la Collagénase et de la Hyaluronidase peuvent ainsi être évaluées par mesure de l'absorbance à 565 nm. L'échantillon est mis en contact avec la solution de Collagénase et Hyaluronidase en même temps que le complexe de substrat des enzymes à 37°C pendant 5 minutes. Le substrat transformé par les enzymes est capable d'activer le chromophore par incubation à 80°C pendant 15 minutes. L'activité de la Collagénase et Hyaluronidase en présence/absence de l'échantillon est alors évaluée par mesure de l'absorbance à 565 nm. La modulation de cette activité est exprimée en pourcentage d'inhibition ou d'activation de l'activité de la Collagénase et Hyaluronidase en l'absence d'actif, c'est-à-dire uniquement en présence du substrat de l'enzyme.

Protocole : Une solution d'enzymes Collagénase de type I et Hyaluronidase est incubée dans son substrat, pendant 5 minutes, en absence ou présence de l'extrait *peregrina* selon l'invention testé. Les solutions sont ensuite mises en contact avec le chromogène Ninhydrin, avant une incubation de 15 minutes à 80°C. A la fin de la période d'incubation, l'activité des enzymes Collagénase et Hyaluronidase avec et sans produit à l'essai ou de référence a été évaluée par mesure de l'absorbance des milieux réactionnels à 565 nm. Pour chaque concentration testée, la modulation de l'activité des enzymes Collagénase et Hyaluronidase par le produit à l'essai est calculée selon la formule suivante.

[Math. 2] Pourcentage de modulation de l'activité des enzyme Collagénase/Hyaluronidase = 100 x [(DO produit à l'essai ou de référence – DO Collagénase/Hyaluronidase seule)/DO Collagénase/Hyaluronidase seule].

Si le résultat est négatif, le pourcentage est exprimé en inhibition des enzymes ; si le résultat est positif, le pourcentage est exprimé en activation des enzymes. Les résultats de l'inhibition des Métalloprotéases sont donnés ci-dessous.

[Tableau 4]

| | | Inhibition versus Contrôle (%) |
|---|---|---|
| Hydrolysat peptidique *peregrina* selon l'invention | 1% | 100 |
| | 0.5% | 100 |
| | 0.1% | 92 |
| | 0.01% | 50 |

Conclusion : L'hydrolysat peptidique *peregrina* selon l'invention engendre une forte inhibition des Métalloprotéases (Collagénase / Hyaluronidase). Il est capable d'inhiber totalement ces Métalloprotéases dès la concentration de 0.5% et a un bon potentiel pour protéger avec une grande efficacité la matrice extra-cellulaire de la peau et, par cette inhibition, il montre un effet anti-vieillissement.

Des tests comparatifs sont reportés ci-après avec l'extrait obtenu selon le brevet FR2946879 de Pierre Fabre dont voici les résultats, selon le même test impliquant simplement la collagénase :

[Tableau 5]

| | Pourcentage | Inhibition versus Contrôle (%) |
|---|---|---|
| Extrait de *Moringa oleifera* selon Brevet Pierre Fabre | 1% | Concentration non compatible avec système d'essai |
| | 0,5% | 4 |
| | 0,1% | 24 |
| | 0,01% | 42 |

Conclusion : l'extrait selon le brevet de Pierre Fabre montre une légère action inhibitrice sur l'activité collagénase en dose-dépendance inverse avec un pic d'inhibition de 42% toutes concentrations confondues contre un pic d'inhibition de 100% pour l'hydrolysat peptidique selon l'invention.

L'activité anti-âge sur ce paramètre apparaît différente et nouvelle comparativement aux effets observés avec l'extrait selon le brevet de Pierre Fabre.

Exemple 4 : Effet de l'hydrolysat peptidique *peregrina* selon l'invention pour inhiber les enzymes Histones Desacétylases (HDACs) et Sirtuine I

L'objectif de cette étude est de démontrer l'activité inhibitrice de l'hydrolysat peptidique *peregrina* selon l'invention sur les enzymes HDACs et Sirtuines I. Une solution tamponnée de HDACs & Sirtuine I réagit avec un substrat pendant 20 minutes à 37°C et le transforme pour former un composé qui se colore en présence d'un développeur après incubation à 37°C pendant 10 minutes. L'activité maximum de désacétylation des Sirtuines peut ainsi être évaluée par mesure de l'absorbance à 405 nm L'extrait *peregrina* selon l'invention ou le produit de référence « Trichostatin A (STA) inhibiteur $1\mu M$ » sont mis en contact avec la solution de Sirtuines en même temps que le substrat de l'enzyme pendant 20 minutes à 37°C, le substrat transformé par l'enzyme est coloré par l'ajout d'un développeur. L'activité désacétylante des HDACs et Sirtuine I en présence de l'actif est alors évaluée par mesure de l'absorbance à 405 nm. La modulation de cette activité est exprimée en pourcentage d'inhibition ou d'activation de l'activité maximum des HDACs & Sirtuine I en absence d'actif, c'est-à-dire uniquement en présence du substrat des enzymes HDACs & Sirtuine I.

Protocole : Une solution d'enzymes Sirtuines est incubée dans son substrat pendant 20 mn en absence (contrôle) ou présence du produit de référence, ou de concentrations croissantes des produits à l'essai. L'hydrolysat peptidique *peregrina* selon l'invention est testé aux concentrations suivantes : 2% ; 1 % ; 0.1 % (VN). A la fin de la période d'incubation, l'activité des enzymes Sirtuines avec et sans produit à l'essai ou de référence a été révélée par coloration à l'aide d'une solution de développeur (10 mn à 37°C) et évaluée par mesure de l'absorbance des milieux réactionnels à 405 nm. Pour chaque concentration testée, la modulation de l'activité désacétylante des enzymes Histones Désacétysases et Sirtuines I par le produit à l'essai est calculée selon la formule suivante.

[Math. 3] Pourcentage de modulation de l'activité des enzymes Sirtuines = 100 x $[(DO_{405}$ produit à l'essai ou de référence) $- (DO_{405}$ HDACs & Sirtuine I seules)]/$DO_{405}$ Sirtuines seules.

Si le résultat est négatif, le pourcentage est exprimé en inhibition de la réaction enzymatique ; si le résultat est positif, le pourcentage est exprimé en activation de la réaction enzymatique. Les résultats de l'inhibition des enzymes Histones Desacétylases (HDACs) sont donnés ci-dessous.

[Tableau 6]

| | pourcentage | Inhibition versus Contrôle (%) |
|---|---|---|
| Hydrolysat peptidique *peregrina* selon l'invention | 2% | 15 |
| | 1% | ns |
| | 0.10% | -18 |

Conclusion : A 2% L'hydrolysat peptidique *peregrina* selon l'invention montre une inhibition significative des HDACs ; cette inhibition signifie la capacité de promouvoir l'autoprotection des cellules de la peau contre la dérive génétique, notamment liée au processus de vieillissement. Ainsi l'extrait apparaît être utile contre l'une des dérives génétiques les plus courantes à la surface de la peau à savoir la fibrose qui se manifeste par l'apparition de « bouton » de chair (protubérance fibrotique). L'extrait pourra avantageusement interférer avec les phénomènes de fibrose à la surface de la peau et prévenir ainsi le vieillissement de la peau.

Exemple 5 : Effet tenseur de l'hydrolysat peptidique *peregrina* selon l'invention.

L'objectif de cette étude est d'évaluer l'effet tenseur de l'actif hydrolysat peptidique peregrina dans un modèle *in vitro* acellulaire de disques de collagène lyophilisé. Des disques de collagène lyophilisé de 8 mm de diamètre ont été utilisés. La contraction de ces disques en réponse aux différents traitements a été mesurée par analyse d'image. Plus la surface des disques de collagène diminue, plus l'effet tenseur des actifs est important. Le produit de référence utilisé dans cette étude est l'albumine sérique bovine à 100 mg/ml.

Protocole : Des disques de collagène de 8 mm de diamètre ont été imbibés avec 40 µl d'eau ultra pure (contrôle), du produit de référence, ou de concentrations croissantes de l'hydrolysat peptidique *peregrina* selon l'invention : 0.05 ; 0.1 et 0.5% (v/v). L'hydrolysat peptidique *peregrina* a été solubilisé directement dans l'eau ultra pure. Des clichés numériques de chacun des disques ont été réalisés à l'aide d'un scanner, avant et 30 minutes après imbition. La mesure de la surface des disques de collagène a été réalisée sur les clichés à l'aide d'un logiciel d'analyse d'images. Les résultats de l'effet tenseur sont donnés ci-dessous.

[Tableau 7]

| | | **Réduction Versus Contrôle (%)** |
|---|---|---|
| Hydrolysat peptidique *peregrina* selon l'invention | 0.5% | 51.1 |
| | 0.1% | 38.4 |
| | 0.05% | ns |

Conclusion : L'hydrolysat peptidique peregrina selon l'invention montre un effet tenseur très significatif à faible concentration (moins de 1%). Ces résultats permettent de démontrer un effet « liftant » et par conséquent un effet antivieillissement à court terme.

Les exemple 2 à 5 permettent de démontrer que l'hydrolysat peptidique *peregrina* selon l'invention a le profil d'un bon actif antivieillissement, à court ou long terme.

Exemple 6 : Effet de l'hydrolysat peptidique *peregrina* selon l'invention pour inhiber l'action de l'endothéline 1

Il a été rapporté ces dernières années que l'endothéline favorise une augmentation de la concentration intracellulaire de calcium dans les mélanocytes épidermiques (cellules de mélanine) pour faciliter la croissance cellulaire à travers le système de transduction du signal intracellulaire. Cette action améliore l'activité de la tyrosinase qui est une enzyme déterminant la vitesse dans la synthèse de mélanine (KADONO, S. et al., 2001, The Rôle of the Epidermal Endothelin Cascade in the Hyperpigmentation Mechanism of Lentigo Senilis, Journal of Investigative Dermatology, 116(4): 571-577). Il a également été rapporté (KAWAHARA N. et al., 2005, About the Component of Snow Tea, Collection of Pharmaceutical, Society of Japan annual convention Subject matter, abstract 30-0823, p. 159) que l'endothéline est un facteur d'activation des mélanocytes produit par les kératinocytes épidermiques, ainsi qu'un facteur important dans la pigmentation induite par la lumière ultraviolette ou la tache sombre sénile (Pang, Y., Geng, J., Oin, Y. et al. Endothelin-1 increases melanin synthesis in an established sheep skin mélanocyte culture. In Vitro Cellular & Developmental Biology - Animal 52, 749-756 (2016) ou HACHIYA, AKIRA et al. Biochemical Characterization of Endothelin-converting Enzyme-1α in Cultured Skin-derived Cells and Its Postulated Role in the Stimulation of Melanogenesis in Human Epidermis.

Journal of Biological Chemistry, Volume 277, Issue 7, 5395 - 540). Ces actions biologiques de l'endothéline suggèrent que des actifs capables d'inhiber l'action de l'endothéline peuvent être utiles pour réduire ou empêcher la production de mélanine ou la pigmentation.

L'endothéline est le vaso-constricteur le plus puissant connu dans le corps humain. D'un autre côté, la diminution de l'endothéline est également connue pour créer un effet vasodilatateur (HIRATA, Y. et al., 1988, Cellular mechanism of action by a novel vasoconstrictor endothelin in cultured rat vascular smooth muscle cells, Biochemical and Biophysical Research Communications, 154(3) : 868-875 ; SHALINKUMAR P. et al., 2008, H2S Médiates the Vasodilator Effect of Endothelin-1 in the Cérébral Circulation. American Journal of Physiology. Heart Circulatory Physiology, 315, p. 1759-1764).

L'objectif est de doser l'endothéline de type 1 dans les cellules endothéliales micro-vasculaires humaines après exposition pendant 24h à l'hydrolysat peptidique *peregrina* selon l'invention.

Protocole : Les cellules endothéliales micro-vasculaires humaines ont été fournies par la société PELOBiotech et mise en culture dans les plaques 96 puits selon les procédures de production du fournisseur. Il s'agit de laisser agir les extraits à différentes concentrations sur les cellules endothéliales à 80% de confluence pendant 24 heures, puis de quantifier l'endothéline 1 à l'aide du kit ELISA PicoKine (EDN1) dans les surnageants cellulaires. Un test de viabilité au préalable est réalisé pour définir les doses non-toxiques à utiliser lors de dosage de l'endothéline 1. Le contrôle négatif est réalisé à l'aide des cellules en milieu de culture sans traitement. Le contrôle positif dans le test de viabilité est le SDS à 0,5%. Toutes les conditions sont préparées dans des milieux de culture, et les cellules sont ensuite incubées à 36.5°C / 5% CO2 pendant 24 heures.

a) Application des solutions à tester sur les cellules endothéliales :
Les produits à tester sont mis en contact avec les cellules endothéliales en sub-confluence dans les plaques 96 puits. Pour chaque concentration le test est réalisé sur 3 puits. Les plaques sont incubées pendant 24 heures ± 1 heure à 36,5°C / 5% CO2.

b) Test de viabilité :
La viabilité cellulaire est évaluée par la méthode MTT sur les cellules après incubation avec les produits. Après 24 heures d'incubation, les surnageants sont récupérés et conservés à -20°C pour les dosages. Les puits sont ensuite rincés 1 fois avec 200 $\mu$L de PBS. Dans chaque puits 50 $\mu$L de solution de MTT à 0,5 mg/ml sont ajoutés : incubation pendant 3 heures à 36.5°C / 5% CO2. Dans chaque puits 100 $\mu$L d'isopropanol sont ajoutés. Après homogénéisation, une lecture de l'absorbance est réalisée à 550 nm. Pour chaque condition, le rapport de la moyenne des densités optiques des cellules sur la moyenne des densités optiques des contrôles négatifs déterminera le taux de viabilité.

c) Dosage de l'endothéline 1 :
Le dosage est réalisé à l'aide du kit ELISA. Les résultats de l'inhibition de l'action de l'endothéline sont donnés ci-dessous.

[Tableau 8]

| | Extrait concentration | Croissance cellulaire versus contrôle (%) | Endothelin 1 versus contrôle (%) | Endothelin 1 versus contrôle (*pg/ml*) |
|---|---|---|---|---|
| Extrait *peregrina* selon l'invention | 2% | +8.46 | -34.90 | -47.09 |
| | 1% | 1.54 | -13.03 | -17.58 |
| | 0.10% | -1.15 | -12.44 | -16.79 |

Conclusion : Le test de viabilité réalisé à la fin de traitement n'a pas montré d'effet toxique pour les concentrations testées.

Le dosage de l'endothéline 1 est réalisé dans les surnageants cellulaires à des concentrations non toxiques. La quantité de l'endothéline 1 pour chaque condition est dosée à l'aide du kit ELISA.

Pour les cellules du contrôle négatif, les valeurs sont de l'ordre de 134.94 pg/ml. Pour les cellules traitées avec différentes concentrations d'extraits, les valeurs sont de 87.85 pg/ml (avec 2% de l'extrait selon l'invention) à 118.15 pg/ml (avec 0.1% de l'extrait selon l'invention), ce qui montre des inhibitions très significatives dès 0.1% de l'extrait selon l'invention avec environ 12.44% d'inhibition de production d'endothéline de type 1 et jusque 34.90% d'inhibition avec 2% de l'extrait selon l'invention.

Les résultats sur l'endothéline et son inhibition dose-dépendante spécifique démontrent que l'hydrolysat peptidique selon l'invention est un puissant éclaircissant de la peau.

Exemple 7 : Effet de l'hydrolysat peptidique *peregrina* selon l'invention pour libérer les acides gras libres.

La lipolyse dans les adipocytes, ou l'hydrolyse du triacylglycérol (TAG) pour libérer les acides gras et le glycérol pour une utilisation par d'autres organes, est une fonction unique du tissu adipeux blanc. La lipolyse dans les adipocytes se produit à la surface des gouttelettes lipidiques cytosoliques, qui ont récemment attiré beaucoup d'attention en tant qu'organites dynamiques, partie intégrante du métabolisme lipidique. L'identification récente de l'AdPLA en tant que phospholipase adipeuse A(2) principale a conduit à la découverte d'une régulation autocrine/paracrine dominante de la lipolyse par la PGE (2). Les mécanismes ci-dessus sont des acteurs clés de la lipolyse et de sa régulation. Des découvertes récentes relient la lipolyse aux altérations ou mutations génétiques et prévoient l'activation de la lipolyse dans les adipocytes comme cible thérapeutique potentielle (AHMADJAN M. et al., 2010, Lipolysis in Adipocytes, The International Journal of Biochemistry and Cell Biology, 42(5): 555-559).

L'objectif de l'étude est d'évaluer la capacité de l'hydrolysat peptidique selon l'invention à augmenter la libération de glycérol dans la culture de cellules humaines (adipocytes). La teneur en glycérol est corrélée à la lipolyse. L'augmentation de la teneur en glycérol implique un effet de lipolyse. L'effet amincissant cosmétique est associé à l'effet lipolyse.

Protocole : Les adipocytes humains sont mis en culture dans les plaques 24 et 12 puits selon les procédures internes. Il s'agit de laisser agir les échantillons à des concentrations définies sur les adipocytes à confluence pendant 24 heures. Un test préalable de viabilité au MTT après 24 heures permet d'évaluer la cytotoxicité et de choisir les concentrations pour le test « effet lipolytique ». Cet « effet lipolytique » est évalué par le dosage du glycérol dans les surnageants après 24 heures d'exposition aux échantillons.

Le contrôle négatif est réalisé à l'aide des cellules en milieu de culture sans traitement. Le contrôle positif pour le test de viabilité est le SDS à 0.5%. Le contrôle positif pour le test « effet lipolytique » est la caféine à 0.05%. Toutes les conditions sont préparées dans des milieux de culture, et les cellules sont ensuite incubées à 36.5°C / 5% $CO_2$ pendant 24 $\pm$ 1 heures.

- Application des solutions à tester sur les adipocytes :

    - Les produits à tester sont mis en contact avec les adipocytes en confluence dans les plaques 24 puits (test de cytotoxicité) et 12 puits (test « effet lipolytique »).
    - Pour chaque concentration, le test est réalisé sur 3 puits.
    - Les plaques sont incubées pendant 24 $\pm$ 1 heures à 36.5°C / 5% $CO_2$.

- Test de viabilité :

    - La viabilité cellulaire est évaluée par la méthode MTT sur les cellules après 24 heures d'incubation avec les produits.
    - Après 24 heures d'incubation, les puits prévus sont rincés 1 fois avec 200$\mu$L de PBS.
    - Dans chaque puits 300 $\mu$l de solution de MTT à 0.5 mg/ml sont ajoutés : incubation pendant 3 heures à 36.5°C / 5% $CO_2$.
    - Dans chaque puits 800 $\mu$l d'isopropanol sont ajoutés.
    - Après homogénéisation, une lecture de l'absorbance est réalisée à 550 nm.
    - Pour chaque condition, le rapport de la moyenne des densités optiques des cellules sur la moyenne des densités optiques des contrôles négatifs déterminera le taux de viabilité.
    - Une valeur seuil de viabilité à 70 % par rapport à la valeur du contrôle négatif est utilisée pour classifier les substances testées comme cytotoxiques ou non cytotoxiques.

- Test « effet lipolytique » :

    - L'effet lipolytique est évalué par le dosage de glycérol dans les milieux de culture après 24 $\pm$ 1 heures d'incubation avec les produits.
    - Les milieux de cultures sont mélangés avec le « free glycerol reagent » selon le protocole du fournisseur.
    - La solution de glycérol est utilisée pour faire la gamme étalon à des concentrations de 0 à 6250 $\mu$M.
    - La lecture de DO est réalisée à 550 nm. Les résultats de la libération des acides gras libres sont donnés ci-dessous.

[Tableau 9]

| | Concentration | Croissance cellulaire versus contrôle (Caféine, %) | l Augmentation de la production de glycérol versus contrôle (%) | Production de glycérol Production ($\mu M$) |
|---|---|---|---|---|
| Hydrolysat peptidique *peregrina* | 2% | -6.16 | +40.00 | 8.32 |
| | 0.5% | -7 | +3.00 | 0.63 |
| | 0.10% | +9.03 | NS | 0 |

Conclusion : L'hydrolysat peptidique selon l'invention a montré qu'il favorisait la libération de glycérol des adipocytes humaines et induisait de ce fait un effet amincissant.

Exemple 8 : Effet de l'extrait *peregrina* selon l'invention pour stimuler la protéine ZAG

L'alpha-2-glycoprotéine de zinc (ZAG) est une glycoprotéine plasmatique qui tire son nom de sa mobilité électrophorétique et de sa capacité à être précipité par les sels de Zn. Le ZAG fait partie de la superfamille des gènes d'immunoglobulines et possède une structure tridimensionnelle hautement homologue aux molécules du CMH de classe I et II. ZAG a été détecté immunohistochimiquement dans les cellules épithéliales sécrétoires normales du sein, de la prostate et du foie, dans les glandes salivaires, bronchiques, gastro-intestinales et sudoripares et dans les épithéliums stratifiés normaux, y compris l'épiderme. L'ARNm de ZAG reste uniformément distribué dans différents types de cellules [Za7]. Du fait de sa production par l'épithélium sécrétoire, le ZAG est présent dans la plupart des sécrétions corporelles et constitue respectivement 2.5% des protéines de la salive et 30% des fluides séminaux.

Les fonctions standard de ZAG ne sont pas claires. Cependant, le ZAG a été isolé de l'urine de patients cancéreux humains atteints de cachexie et peut fonctionner comme un facteur de mobilisation des lipides. Le ZAG purifié extrait du sérum humain ou de souris, ou extrait de l'urine humaine chez les patients cancéreux, induit une lipolyse, entraînan la libération de glycérol. Ce dernier augmente également l'utilisation des lipides dans les adipocytes humains et de souris (HIRAI K. et al., 1998, Biological Evaluation of a Lipid-Mobilizing Factor Isolated from the Urine of Cancer Patients, Cancer Research, 58(11) : 2359-2365). Le ZAG active une activité d'adénylate cyclase dépendante du guanosine triphosphate (GTP) sur les membranes des adipocytes, augmentant les niveaux d'adénosine monophosphate cyclique (AMPc) cellulaire. Cela peut potentiellement conduire à l'activation de multiples voies cellulaires.

Pour approfondir les propriétés biologiques de ZAG, des transfectants stables du ZAG humain recombinant (rh) ont été créés dans la lignée cellulaire de mélanome murin B16F10. L'effet de la transfection de ZAG sur la production de mélanine a été déterminé *in vitro* et *in vivo.* Enfin, l'effet de ZAG sur l'expression et l'activité de la tyrosinase a été déterminé. Dans l'ensemble, ces études montrent que le ZAG inhibe la production de mélanine dans les mélanocytes normaux et malins. Les mécanismes comprennent des effets post-transcriptionnels sur la protéine tyrosinase, avec le potentiel d'effets indirects supplémentaires. Ces études ont permis d'identifier une fonction biologique du ZAG jusque-là inconnue et ont rendu possible une méthode de modulation de la production de mélanine, empêchant et/ou diminuant ainsi la pigmentation de la peau et des cheveux due à l'augmentation de la production de mélanine (HALE L., Method of Modulating Melanin Production, United States Patent No. US7,803,750 B2, 2010) et (GHADA F. M. et al., 2015, Highlights in Pathogenesis of Vitiligo, World Journal of Clinical Cases 3(3): 221-230).

L'objectif de cette étude est d'évaluer la capacité de l'hydrolysat peptidique *peregrina* selon l'invention à augmenter la libération de ZAG dans la culture de cellules humaines (kératinocytes). La teneur en ZAG est à corréler avec un effet blanchissant, un effet amincissant, un effet anti-fibrotique et un effet apaisant.

Protocole : Les kératinocytes humains normaux sont isolés à partir de prépuces et mis en culture dans les plaques 24 et 96 puits selon les procédures internes.

Il s'agit de laisser agir les échantillons à des concentrations définies sur les kératinocytes à 80% de confluence pendant 48 heures, puis de quantifier les ZAG à l'aide du kit ELISA dans les surnageants cellulaires.

Un test préalable de viabilité est réalisé pour définir les doses non-toxiques à utiliser lors de dosage des ZAG.

Le contrôle négatif est réalisé à l'aide des cellules en milieu de culture sans traitement.

Le contrôle positif pour le test de viabilité est le SDS à 0.5%.

Toutes les conditions sont préparées dans des milieux de culture, et les cellules sont ensuite incubées à 36.5°C / 5% CO2 pendant 24 heures pour le test de viabilité et 48 heures pour le dosage des ZAG.

• Application des solutions à tester sur les kératinocytes :

- Les produits à tester sont mis en contact avec les kératinocytes en sub-confluence dans les plaques 24 et 96 puits.
- Pour chaque concentration, le test est réalisé sur 3 puits.
- Les plaques sont incubées pendant 24 heures et 48 heures à 36.5°C / 5% CO2.

- Test de viabilité :

    - La viabilité cellulaire est évaluée par la méthode MTT sur les cellules après incubation avec les produits.
    - Après 24 et 48 heures d'incubation, les puits prévus sont rincés 1 fois avec 200 µL de PBS.
    - Dans chaque puits 50 µl de solution de MTT à 0.5 mg/ml sont ajoutés : incubation pendant 3 heures à 36.5°C / 5% CO2.
    - Dans chaque puits 100 µl d'isopropanol sont ajoutés.
    - Après homogénéisation, une lecture de l'absorbance est réalisée à 550 nm.
    - Pour chaque condition, le rapport de la moyenne des densités optiques des cellules sur la moyenne des densités optiques des contrôles négatifs déterminera le taux de viabilité.

- Dosage des protéines ZAG :

    - Après 48 heures d'incubation, tous les surnageants sont récupérés et conservés à -20°C pour les dosages.
    - Le dosage est réalisé à l'aide d'un kit ELISA. Les résultats du dosage de ZAG sont donnés ci-dessous.

[Tableau 10]

| | Concentration | Croissance cellulaire versus contrôle (%) | ZAG versus contrôle (%) | ZAG versus contrôle (ng/ml) |
|---|---|---|---|---|
| Hydrolysat peptidique peregrina | 2% | -17.88 | +337.80 | 0.390 |
| | 1% | -12.54 | +195.73 | 0.157 |
| | 0.1 | -3.88 | +151.83 | 0.085 |

Conclusion : L'hydolysat peptidique *peregrina* peut augmenter de manière significative la production de ZAG, avec une bonne dépendance à la dose et une faible toxicité sur les cellules humaines. Il a de ce fait un effet blanchissant, un effet amincissant, un effet anti-fibrotique et un effet apaisant basé sur sa capacité à augmenter significativement la ZAG.

Exemple 9 : Effet de l'hydrolysat peptidique *peregrina* selon l'invention pour inhiber la production de mélanine

La pigmentation de la peau est un processus complexe qui, dans l'épiderme comme dans les follicules pileux, commence par la synthèse de mélanine dans les mélanosomes à l'intérieur des mélanocytes, suivie du transfert des mélanosomes aux kératinocytes environnants, qui à leur tour transportent le pigment et éventuellement le dégrade. Chez l'homme, toute la population mélanocytaire est localisée dans les follicules pileux et dans la couche basale de l'épiderme. Quelle que soit leur localisation dans la peau, les mélanocytes ont une origine embryologique commune, la crête neurale dont ils dérivent sous forme de mélanoblastes (cellules non pigmentées). Il existe deux types de mélanine dans les cellules épidermiques, l'eumélanine, un pigment brun-noir, et la phéomélanine, un pigment jaune-rouge. Dans les mélanocytes, les eumélanosomes et les phéomélanosomes coexistent. La tyrosinase est l'enzyme clé qui régule les premières étapes de la synthèse de la phéomélanine et de l'eumélanine: la conversion de la L-tyrosine en L-3,4-dihydroxyphénylalanine (L-DOPA) et l'oxydation de ce composé en dopaquinone. À partir de la dopaquinone, les voies de synthèse diffèrent pour l'eumélanine et la phéomélanine. Le rôle principal de la mélanine est de protéger la peau des effets nocifs des rayons UV, empêchant ainsi le développement d'un cancer de la peau (BRENNER M. et al., 2008, The Protective Rôle of Melanin Against UV damage in human skin, Photochemistry and Photobiology 84(3): 539-549).

L'objectif est de regrouper l'ensemble des données utilisées, ainsi que les résultats obtenus, pour la réalisation du test de modulation de la mélanine sur les mélanocytes humains après exposition pendant 5 jours à l'extrait *peregrina* selon l'invention.

Protocole : Les mélanocytes humains sont mis en culture dans les plaques 96 et 24 puits.

Il s'agit de laisser agir l'extrait *peregrina* selon l'invention à des concentrations de 5% ; 2% ; 1% et 0.1% sur les mélanocytes à confluence pendant 5 jours. Un pré-test de viabilité au MTT après 24 heures permet d'évaluer la cytotoxicité et de choisir les concentrations pour le test de modulation de la mélanine. Cette modulation est évaluée par le dosage de la mélanine dans les lysats cellulaires après 5 jours d'exposition aux extraits. Le contrôle négatif est réalisé à l'aide des cellules en milieu de culture sans traitement. Le contrôle positif pour le test de viabilité est le SDS à 0.5%. Pour le test de la modulation de la mélanine des milieux avec et sans $\alpha$-MSH sont utilisés comme contrôles négatifs.

Toutes les conditions sont préparées dans des milieux de culture, et les cellules sont ensuite incubées à 36.5°C / 5% CO2 pendant 24 heures pour le test de cytotoxicité et 5 jours pour le dosage de mélanine.

a) Application des solutions à tester sur les mélanocytes : Les concentrations à tester sont mises en contact avec les mélanocytes en confluence dans les plaques 96 (test de cytotoxicité) et 24 (dosage de mélanine) puits. Pour chaque concentration le test est réalisé sur 3 puits. Les plaques sont incubées pendant 24 $\pm$ 1 heures et 5 jours à 36.5°C / 5% CO2.

b) Test de viabilité : La viabilité cellulaire est évaluée par la méthode MTT sur les cellules après 24 heures d'incubation avec les produits. Après 24 heures d'incubation, les puits prévus sont rincés 1 fois avec 200 $\mu$L de PBS. Dans chaque puits 50 $\mu$l de solution de MTT à 0.5 mg/ml sont ajoutés et l'incubation est effectuée pendant 3 heures à 36.5°C / 5% CO2. Dans chaque puits 150 $\mu$l d'isopropanol sont ajoutés. Après homogénéisation, une lecture de l'absorbance est réalisée à 550 nm. Pour chaque condition, le rapport de la moyenne des densités optiques des cellules sur la moyenne des densités optiques des contrôles négatifs déterminera le taux de viabilité.

Une valeur seuil de viabilité à 70 % par rapport à la valeur du contrôle négatif est utilisée pour classifier les substances testées comme cytotoxiques ou non cytotoxiques. Une classification « non cytotoxique » est donnée sur les résultats in vitro pour une viabilité > 70% et une classification « cytotoxique » pour une viabilité $\leq$ 70%.

La concentration à 5% de l'extrait selon l'invention s'est montrée cytotoxique à 5% dans les conditions du test. Les concentrations 2%, 1% et 0.1% sont donc utilisées pour le test de modulation de la mélanine. La quantité de mélanine présente dans les cellules est dosée après la lyse cellulaire. Les résultats de l'inhibition de la production de mélanine sont donnés ci-dessous.

[Tableau 11]

| | Extrait concentration | Croissance cellulaire versus contrôle (%) | Inhibition Mélanine versus contrôle (%) | Contenu en Mélanine ($\mu g$/ml) |
|---|---|---|---|---|
| Hydrolysat peptidique selon l'invention | 2% | -24.48 | +16.50 | 152 |
| | 1.0% | -17.15 | +55.00 | 82 |
| | 0.10% | -18.06 | +71.40 | 52 |

Conclusion : L'hydrolysat peptidique peregrina selon l'invention inhibe la production de mélanine in cellulo ce qui lui confère un effet dépigmentant et une propriété blanchissante de la peau.

Exemple 10: Effet de l'hydrolysat peptidique peregrina selon l'invention sur la modulation du dosage de DKK1 et DKK3

L'implication des interactions entre les mélanocytes et les fibroblastes dans la régulation de la mélanogenèse est bien connue et a été étudiée de manière intensive. Bien que ces interactions ne soient pas encore parfaitement comprises, elles sont à l'origine de la "blancheur" des zones palmo-plantaires et sont désormais utilisées en cosmétique pour le développement de produits dépigmentants Yamaguchi et collègues (YAMAGUCHI Y. et al., 2004, Mesenchymal-Epithelial Interactions in the Skin: Increased Expression of Dickkopf by Palmoplantar Fibroblasts Inhibits Melanocyte Growth and Différentiation, Journal of Cell Biology 165(2): 275-285) ont démontré qu'un messager soluble produit par les fibroblastes des régions palmo-plantaires, était capable de modifier le programme de différenciation des mélanocytes de ces régions, conduisant à une diminution de la production de mélanine. Ce messager a été identifié par l'équipe comme une protéine nommée Dikkopf-1 (DKK-1).

Les voies de signalisation utilisées par DKK-1 pour produire ces résultats sont bien identifiées aujourd'hui. Grâce à son action antagoniste sur le récepteur Wnt, DKK-1 est en effet capable de "shunter" les voies de signalisation intracellulaires activées par la $\beta$-caténine, généralement responsables de la régulation des gènes impliqués dans la mélanogenèse. Yamaguchi et collègues ont également démontré que DKK-3, une molécule proche de DKK-1, mais sans effet sur le récepteur Wnt, pourrait jouer un rôle régulateur sur l'effet de DKK-1. En fait, plus la quantité de DKK-3 au voisinage de ce récepteur Wnt est importante, plus les interactions entre DKK-1 et ce récepteur sont faibles. L'augmentation de DKK3 réduit les effets inhibiteurs de DKK-1 sur la mélanogenèse. Les travaux de Yamaguchi et collègues (cf. *supra*) suggèrent que l'identification d'agents ayant une influence sur le rapport DKK1 / DKK3 dans des cultures de fibroblastes dermiques humains normaux d'origine non palmo-plantaire permettrait de contrôler la production de mélanine à partir de mélanocytes humains normaux non palmo-plantaires

L'objectif de cette étude est d'évaluer l'effet du composé dénommé « Hydrolysat peptidique *peregrina* » sur la synthèse et la libération de DKK-1 dans un modèle composé de fibroblastes humains normaux en culture monocouche.

Protocole : Les cellules de fibroblastes humains sont obtenues auprès d'un donneur âgé de 68 ans. Pour réaliser les expériences, les fibroblastes sont cultivés en monocouche jusqu'à atteindre la confluence. La dexaméthasone à 100 nm a été utilisée comme inducteur de référence de la synthèse et de la libération de DKK-1.

Les disques cutanés ont été incubés pendant 48 heures en absence (contrôle) ou en présence de produit de référence ou de produit à tester : « Hydrolysat peptidique *peregrina* » : 0.01; 0.1 et 0.5% (v/v).

À la fin de l'incubation, les milieux d'incubation ont été retirés pour effectuer la mesure de libération de DKK-1.

Le composé d'essai « Hydrolysat peptidique *peregrina* » a été dilué directement dans le milieu d'incubation afin d'atteindre les différentes concentrations décrites ci-dessus.

À la fin de la période d'incubation de 48 heures, le DKK-1 libéré dans les milieux d'incubation a été quantifié à l'aide d'un kit ELISA sensible et spécifique.

À la fin de la période d'incubation, les protéines contenues dans les lysats cellulaires ont été quantifiées à l'aide d'une méthode spectro-colorimétrique (méthode Bradford).

Les résultats sont exprimés en ng de DKK-1 par mg de protéines (moyenne ± S.D.).

Le niveau de signification entre le « contrôle » et le « produit de référence » a été évalué à l'aide d'un test de Student (*: $p < 0.05$).

Le niveau de signification entre le « contrôle » et le « produit d'essai » a été évalué par une analyse de variance à un facteur (ANOVA unidirectionnelle) suivie d'un test de Holm-Sidak (*: $p < 0.05$).

Dans nos conditions expérimentales, le produit de référence nommé « Dexamethasone », testé à 100 nm, a significativement augmenté le DKK-1 libéré de 181.8% ($p < 0.01$) par rapport à « Contrôle ». Les résultats sur la modulation du dosage de DKK1 sont donnés ci-dessous.

[Tableau 12]

|  | Concentration | Croissance cellulaire versus contrôle (%) | DKK1 versus contrôle (%) |
|---|---|---|---|
| Hydrolysat peptidique *peregrina* | 0.5% | +1.9 | +131.50 |
|  | 0.1% | ca. 0 | +32.30 |
|  | 0.05% | -0.6 | +26.10 |
| Etude sur DKK3 |  |  |  |

Le but de cette étude est d'évaluer l'effet de composés appelés « Hydrolysat peptidique *peregrina* » sur la synthèse et la libération de DKK-3 dans un modèle composé de fibroblastes humains normaux en culture monocouche.

Les cellules de fibroblastes humains ont été obtenues auprès d'un donneur âgé de 68 ans.

Pour réaliser les expériences, les fibroblastes ont été cultivés en monocouche jusqu'à atteindre la confluence.

Les cellules de fibroblastes humains ont été obtenues d'un donneur de 68 ans.

Pour réaliser les expériences, les fibroblastes ont été cultivés en monocouche jusqu'à atteindre la confluence.

À la fin de la période d'incubation de 48 heures, le DKK-3 libéré dans les milieux d'incubation a été quantifié à l'aide d'un kit ELISA sensible et spécifique.

À la fin de la période d'incubation, les protéines contenues dans les lysats cellulaires ont été quantifiées à l'aide d'une méthode spectro-colorimétrique (méthode Bradford).

Les résultats sont exprimés en ng de DKK-3 par mg de protéines (moyenne ± S.D.).

Le niveau de signification entre le « contrôle » et le « produit de référence » a été évalué à l'aide d'un test de Student (*: $p < 0.05$).

Le niveau de signification entre le « contrôle » et le « produit d'essai » a été évalué par une analyse de variance à un facteur (ANOVA unidirectionnelle) suivie d'un test de Holm-Sidak (*: $p < 0.05$). Les résultats sur la modulation du dosage de DKK3 sont donnés ci-dessous.

[Tableau 13]

| | Concentration | Croissance cellulaire versus contrôle (%) | DKK3 versus contrôle (%) |
|---|---|---|---|
| Hydrolysat peptidique *peregrina* | 0.5% | 101.9 | -20.7 |
| | 0.1% | env. 100 | -8 |
| | 0.05% | 99.4 | -3.9 |

Conclusion : L'hydrolysat peptidique *peregrina* selon l'invention peut augmenter considérablement la production de DKK1, et tend à réduire la production de DKK3 dans les cellules humaines. Il a une forte capacité à diminuer la pigmentation de la peau grâce au principe d'inhibition palmo-plantaire, par sa capacité à augmenter considérablement DKK1 et diminuer significativement DKK3, ce qui augmente le ratio DKK1/DKK3. L'augmentation de ce ratio conduit à limiter la voie de différenciation pré-mélanocyte / mélanocyte ; ainsi le nombre de mélanocytes opérationnel diminue dans le temps, réduisant la capacité de la peau à se teinter.

Exemple 11 : Effet de l'hydrolysat peptidique *peregrina* selon l'invention pour préserver l'ADN

La dynamique de la longueur des télomères est très importante pour la régulation de la durée de vie réplicative dans les cellules, en particulier chez les espèces à longue durée de vie. Le raccourcissement des télomères et l'activité de la télomérase sont des facteurs importants dans le vieillissement et la tumorigenèse (SHAY, J. W., 2005, Senescence an Immortalization: Role of Telomeres and Telomerase, Carcinogenesis 26(5) : 867-874). Les télomères sont des séquences nucléotidiques complexes qui recouvrent l'extrémité des chromosomes de la dégradation, de la fusion-recombinaison indésirable, de l'activation inappropriée de la réponse aux dommages de l'ADN. Ils jouent également un rôle essentiel dans la division cellulaire et la stabilité des chromosomes. Il existe de plus en plus de preuves que la stabilité des télomères peut être affectée par l'exposition professionnelle et environnementale, car certains de ces facteurs ont été associés à une augmentation de l'inflammation, du stress oxydatif, des dommages à l'ADN, de l'aberration chromosomique et des altérations épigénétiques. Les télomères extrêmement courts et longs ont été associés à des maladies neurodégénératives, cardiovasculaires (MCV) et au risque de cancer.

La télomérase est une ribonucléoprotéine qui catalyse l'addition de répétitions télomériques aux extrémités des télomères. Les télomères sont de longs tronçons de séquences répétées qui coiffent les extrémités des chromosomes et sont censés stabiliser le chromosome. Chez l'homme, les télomères ont généralement une longueur de 7 à 10 kb et comprennent plusieurs répétitions de la séquence -TTAGGG-.

La télomérase n'est pas exprimée dans la plupart des cellules adultes et la longueur des télomères diminue avec les cycles de réplication successifs. Après un certain nombre de cycles de réplication, le raccourcissement progressif des télomères fait entrer les cellules dans une phase de crise télomérique, qui à son tour conduit à la sénescence cellulaire. Certaines maladies sont associées à une perte télomérique rapide, entraînant une sénescence cellulaire prématurée. Il a été démontré que l'expression du gène codant pour la protéine de télomérase humaine dans les cellules humaines (BLASCO M., 2007, Telomere Length, Stem Cells and Aging, Nature Chemical Biology 3, p. 640-649) confère un phénotype immortel, probablement en contournant la voie de sénescence naturelle des cellules. De plus, il a été démontré que l'expression du gène de la télomérase dans les cellules vieillissantes avec de courts télomères produit une augmentation de la longueur des télomères et restaure un phénotype généralement associé aux cellules plus jeunes.

L'objectif de cette étude est d'évaluer l'effet du composé dénommé « Hydrolysat peptidique *peregrina* » sur le raccourcissement des télomères dans un modèle composé de fibroblastes humains normaux en culture monocouche. Il est bien connu que le télomère correspond à une horloge biologique. La longueur des télomères diminue progressivement avec les divisions cellulaires, entraînant finalement une incapacité de réplication de la cellule. La mesure de la longueur des télomères a été effectuée en utilisant la PCR quantitative et en comparaison avec la longueur des télomères entre les cellules aux passages 2 et 5.

Protocole : Des cellules de fibroblastes humains ont été obtenues auprès d'un donneur de 44 ans. Pour effectuer les expériences, des cellules ont été utilisées au passage 2 et 5. Les fibroblastes ont été cultivés pendant 3 passages consécutifs en absence (contrôle) ou en présence d'une concentration croissante à tester de l'hydrolysat peptidique *peregrina*: 0.01; 0.1 et 0.5% (v/v).

Préparation du composé d'essai : Le composé d'essai « Hydrolysat peptidique *peregrina* » a été dilué directement dans le milieu d'incubation afin d'atteindre les différentes concentrations décrites ci-dessus.

A la fin de l'incubation, les cellules ont été trypsinisées. L'ADN a été extrait des cellules à l'aide d'un kit d'extraction d'ADN dédié. L'ADN a été quantifié par nanodrop.

La longueur des télomères a été mesurée par PCR quantitative (q-PCR). Pour chaque échantillon, la variation de la longueur des télomères a été mesurée par quantification relative en utilisant le gène SCR (référence à copie unique) comme gène de référence. Pour chaque échantillon, une q-PCR est effectuée en utilisant un jeu d'amorces de télomères qui reconnaît et amplifie les séquences de télomères et une seconde q-PCR est effectuée en utilisant le jeu d'amorces SCR qui reconnaît et amplifie une région de 100 pb sur le chromosome 17 humain et sert de référence pour la normalisation des données.

Les résultats sont exprimés en unités relatives correspondant à la longueur des télomères par rapport aux cellules au passage 2 (moyenne $\pm$ S.D.). Le niveau de signification entre « contrôle » aux passages 2 et 5 a été évalué à l'aide d'un test de Student t (*p <0.05). Le niveau de signification entre « contrôle » et « composé d'essai » a été évalué indépendamment pour chaque produit par une analyse de variance à un facteur (ANOVA unidirectionnelle) suivie d'un test Holm-Sidak (*: p <0.05).

Résultats : Dans nos conditions expérimentales, l'hydrolysat peptidique *peregrina* testé à 0.05%, 0.1% et 0.5% (v / v), a significativement diminué le raccourcissement des télomères des fibroblastes humains normaux.

Raccourcissement des télomères : inhibition (en comparaison avec le contrôle) à 0.05% (v / v) + 8.9% (p <0.05) et à 0.1% (v / v) + 15.1% (p <0.01) et à 0.5% (v / v) + 16.6% (p <0.01).

[Tableau 14]

|  | Concentration | Croissance cellulaire versus contrôle (%) | Longueur des télomères versus contrôle (%) |
|---|---|---|---|
| Hydrolat peptidique *peregrina* | 0.5% | +1.9 | +16.60 |
|  | 0.1% | ca. 0 | +15.10 |
|  | 0.05% | -0.6 | +8.90 |

Conclusion : Dans un contexte de multiplication ou de division normale des cellules humaines, l'hydrolysat peptidique *peregrina* selon l'invention démontre une capacité à augmenter considérablement la longueur des télomères. Les télomères sont des bouchons impliqués dans la protection du matériel ADN ; l'augmentation de la taille des télomères préserve l'intégrité du matériel ADN au fil du temps. L'augmentation de la longueur des télomères est corrélée à la capacité de préserver le matériel d'ADN. L'hydrolysat peptidique *peregrina* pouvant augmenter cette longueur, est donc impliqué dans la préservation du matériel génétique humain (ADN).

Exemple 12 : Tests comparatifs de différents produits sur l'hydratation à la surface de la peau

L'étude en cornéométrie permet la mesure de l'effet hydratant d'un produit à la surface de la peau. L'appareil utilisé est un cornéomètre doté d'une sonde mesurant notamment l'humidité de la peau par impédancemétrie. Un Panel composé de 6 personnes (2 hommes et 4 femmes) a testé dans des conditions similaires d'hydrométrie et de température sur les faces internes de leur avant-bras. La face interne de l'avant-bras gauche porte les zones 1 et 2, la face interne de l'avant-bras droit porte les zones 3 et 4.

La zone 1 correspond à la zone d'application du gel hydratant de référence ; la zone 2 correspond à la zone d'application du gel de référence + 2% de l'hydrolysat peptidique selon l'invention ; la zone 3 correspond à la zone d'application du gel de référence + 2% de l'hydrolysat peptidique avec *Moringa oleifera ;* la zone 4 correspond à la zone d'application du gel de référence + 2% de Purisoft® selon brevet FR3076460 de de BASF Beauty Care Solutions.

La quantité de produit appliquée sur chaque zone est la même, et les temps de mesures sont les mêmes pour chacune des zones. Une première prise de valeur est faite avant application du produit sur chaque zone de sorte à connaître l'état zéro d'humidité de la peau. Ensuite, pour chaque zone et chaque produit, une première mesure est conduite 5 minutes après l'application du produit sur la zone, toujours par le même opérateur. Enfin une dernière mesure est conduite 30 minutes après la seconde mesure ci-dessus, toujours par le même opérateur.

La combinaison des différences observées avant et après application ne suit pas une loi normale étudiée en ANOVA (analyse la variance). La valeur obtenue par différence de la mesure avant et après est donc le paramètre étudié. Les résultats obtenus constituent une tendance qualitative et non quantitative des valeurs obtenues :

[Tableau 15]

| Zone | Moyenne différences estimées |
|---|---|
| 1 = référence (placebo) | 1.286 |
| 2 = réf. + hydrolysat peptidique selon invention 2% | 1.190 |

(suite)

| Zone | Moyenne différences estimées |
|---|---|
| 3 = Réf. + hydrolysat peptidique de M. *oleifera* 2% | -0.214 |
| 4 = réf. + extrait Purisoft® BASF 2% | -0.667 |

Conclusion : La zone 2 présente une valeur d'hydratation positive pour la peau entre avant et après l'application du produit contenant l'extrait selon l'invention, tandis que les zones 3 et 4 contenant les extraits de *Moringa oleifera* selon 2 modes d'extraction, présentent des valeurs négatives, c'est-à-dire que la peau est moins hydratée après qu'avant application des produits. L'hydrolysat peptidique selon l'invention permet de maintenir le bénéfice d'une hydratation dans un produit d'application topique ce qui distingue l'hydrolysat peptidique peregrina selon l'invention des autres extraits testés. La figure 1 représente les résultats obtenus.

Exemple 13 : Formulation d'un produit maquillant

[Tableau 16]

| Ingrédients | % |
|---|---|
| Eau | QSP |
| Caprylic / Capric triglycéride | 19.0000 |
| Gomme Acacia senegal | 7.0000 |
| Charcoal | 6.0000 |
| Glycérine | 5.0000 |
| Propanediol | 5.0000 |
| Bentonite | 3.1500 |
| Cetearyl glucoside | 3.0000 |
| Cetearyl alcohol | 3.0000 |
| Benzyl alcohol | 1.0000 |
| Hydrolysat peptidique *peregrina* selon invention | 2.0000 |
| Gomme Cellulose | 0.8000 |
| Gomme Xanthane | 0.1750 |
| Acide Citrique | 0.1750 |

Exemple 14 : Formulation d'un produit lavant

[Tableau 17]

| Ingrédients | % |
|---|---|
| Eau | QSP |
| Sodium coco-sulfate | 5.0000 |
| Sodium cocoyl isethionate | 4.0000 |
| Bentonite | 3.7800 |
| Caprylic / Capric triglycéride | 2.0000 |
| Hydrolysat peptidique *peregrina* selon l'invention | 1.0000 |

(suite)

| Ingrédients | % |
|---|---|
| Gluconolactone | 0.7500 |
| Sodium benzoate | 0.5450 |
| Parfum | 0.5000 |
| Gomme Xanthane | 0.2700 |
| Sodium stearoyl glutamate | 0.2250 |
| Acide Citrique | 0.2250 |
| Calcium gluconate | 0.0050 |

Exemple 15 : formulation d'un produit de soin

[Tableau 18]

| Ingrédient | % |
|---|---|
| Eau | QSP |
| Caprylic / Capric triglycéride | 18.0000 |
| Bentonite | 4.2000 |
| Cetearyl alcohol | 1.5000 |
| Hydrolysat peptidique *peregrina* selon invention | 2.0000 |
| Gluconolactone | 0.7500 |
| Sodium benzoate | 0.5450 |
| Gomme Xanthane | 0.5000 |
| Parfum | 0.5000 |
| Sodium stearoyl glutamate | 0.2500 |
| Acide Citrique | 0.2500 |
| Calcium gluconate | 0.0050 |

Exemple 16 : comprimé relaxant ou amincissant de 1 g : 2% extrait sec selon l'invention (contenant 60% d'hydrolysat peptidique sur un support d'inuline) + 47% carbonate de calcium contenant 200 UI de vitamine D + 25% gluconate de magnésium + 23% inuline + 3% stéarate de magnésium).

Exemple 17 : Poudre amincissante en gélule de 750 mg contenant 200 mg de caféine, 200 mg d'extrait sec selon l'invention (contenant 60% d'hydrolysat peptidique sur un support d'inuline), 200 mg de chitosane et 150 mg de carbonate de calcium.)

Exemple 18 : Poudre coupe-faim & minceur en gélule de 750 mg contenant 200 mg de caféine, 200 mg d'extrait sec selon l'invention (contenant 60% d'hydrolysat peptidique sur un support d'inuline), 200 mg de chitosane et 150 mg de Konjac (glucomannane).

Exemple 19 : Spray relaxant sublingual contenant 2% d'extrait selon l'exemple 1. 1% teinture mère de *Rhodiola,* 1% Teinture mère de Mélisse, 1% teinture mère de Valériane, 1% teinture mère d'Aubépine, 2% d'extrait de prêle, 0.15% d'acide salycilique, 7% de sorbitol, et QSP en eau).

Exemple 20 : Crème anti-rides contenant 2% d'extrait hydrolysé selon l'exemple 1

[Tableau 19]

| Ingrédients | % | INCI |
|---|---|---|
| Eau | 70.95 | Aqua |
| Acide salicylique | 0.15 | Salicylic acid |
| Benzoate de sodium | 0.5 | Sodium benzoate |
| Frametime CXG | 3.2 | Bentonite & Xanthan gum & Sodium stearoyl glutamate & Citric acid |
| Gomme de xanthane FF | 0.2 | Xanthan gum |
| Glycérine végétale - Palmera G995E | 3.5 | Glycerin |
| Alcool cétostéarylique 50/50 | 1 | Cetearyl alcohol |
| DUB MCT 5545 | 18 | Caprylic / Capric triglycéride |
| Parfum frangipanier DIV/02356 | 0.5 | Parfum |
| Extrait hydrolysé *Peregrina* | 2 | Moringa *peregrina* hydrolyzed seed extract |

Exemple 21 : Tests toxicologiques de l'hydrolysat selon l'invention

Préparation de l'hydrolysat peptidique conformément à l'exemple 1 : Des graines non décortiquées de *Moringa peregrina* (Forssk.) Fiori récoltées à maturité du fruit ont été séchées pour obtenir un taux d'humidité interne inférieur à 8% et préférentiellement autour de 6%, puis pressées avec une presse mécanique à vis sans fin, de sorte à séparer l'huile du reste de la graine pour obtenir d'une part l'huile vierge et d'autre part un tourteau. Le tourteau est alors isolé sous forme de boudins prédécoupés en morceau de 1 à 2 cm. En suivant le protocole décrit à l'exemple 1, on obtient l'extrait liquide que l'on utilise pur dans les tests qui suivent.

1. Détermination de l'activité mutagène sur la souche bactérienne *Salmonella typhimurium* (TA 100) - Test de mutation bactérienne inverse

Le test s'est déroulé en 3 phases principales :

- Une expérience préliminaire est réalisée afin d'évaluer la cytotoxicité de l'élément à tester et de sélectionner la gamme de doses pour les expériences ultérieures,
- Une première expérience de génotoxicité (Test 1), avec et sans activation métabolique, avec incorporation directe du système de test et du test (ou des contrôles) sur gélose minimale, sur la plage de doses définie par l'étude préliminaire,
- Une deuxième expérience (Test 2), avec pré-incubation du système de test et de l'élément de test (ou des contrôles), avec et sans activation métabolique, avec des niveaux de dose définis par le responsable de l'étude après analyse des résultats de la première expérience. Cette seconde expérience a été réalisée afin de confirmer ou de compléter les résultats de la première, en particulier lorsque des résultats équivoques ou négatifs ont été obtenus.

Les dilutions de l'extrait selon l'exemple 1 ont été préparées dans l'eau pour réaliser le test de cytotoxicité.

Ce dernier a été réalisé sur la souche *Salmonella typhimurium* TA100 aux concentrations de 5000, 1600, 500, 160 et 50 µg / plaque, avec et sans S9-Mix.

Les réactifs utilisés pour la préparation de S9-Mix ont été préparés selon les instructions suivantes :

[Tableau 20]

| | Concentration finale |
|---|---|
| MgCl$_2$ (0.4 M) + KCl (1.65 M) | 8 mM + 33 mM |
| Glucose 6 Phosphate (0.2 M) | 5 mM |
| NADP (0.1 M) | 4 mM |
| Tampon Phosphate pour S9-Mix (pH 7.4 - 0.2 M) | 0.1 M |

26

(suite)

|  | Concentration finale |
|---|---|
| S9 fraction | 10% |
| Eau | Ajuster pour concentration finale |

Les bactéries ont été exposées à l'extrait de test avec et sans système d'activation métabolique. Le système métabolique utilisé est une fraction post-mitochondriale améliorée par cofacteur (S9). Cette fraction S9, une fraction microsomale d'homogénat de foie de rat Sprague Dawley traité avec un inducteur enzymatique, est préparée selon MARON, D.M. ET AMES, B.N. (1983) et a été fournie par MOLTOX TM. Elle est conservée à une température inférieure à -70°C. La fraction microsomale S9 a été utilisée à la concentration de 10% dans S9-Mix. Le protocole appliqué a été le suivant :

- Dans 3 tubes d'hémolyse, a été introduit :

  - dosage sans activation métabolique:

    • 0.1 ml des différentes concentrations des éléments de test.
    • 0.5 ml de tampon phosphate stérile 0,2 M, pH 7,4,
    • 2 ml de top agar pour *S. typhimurium,*
    • 0.1 ml d'inoculum bactérien (TA100).

  - dosage avec activation métabolique:

    • 0.1 ml des différentes concentrations des éléments de test,
    • 2 ml de top agar pour *S. typhimurium,*
    • 0.1 ml d'inoculum bactérien (TA100),
    • 0.5 ml de S9-Mix.

    - Mélanger et verser sur la surface de la gélose de fond préalablement répartie dans des boîtes de Pétri.
    - Incuber à 37 ° C $\pm$ 2 ° C pendant 48 à 72 heures.

Ces dosages ont été réalisés pour chaque test : test préliminaire de cytotoxicité, test 1 et test 2. Le contrôle non traité, les contrôles négatifs et les contrôles positifs réalisés au cours de la méthode de pré-incubation ont été incubés pendant 20 à 30 minutes à 37 ° C $\pm$ 2 ° C avant de verser la gélose supérieure.

Le protocole appliqué a été le suivant :

- Dans 4 fractions de 2 ml d'agar top pour *S. typhimurium,* introduire :

  ◦ 0.1 ml de tampon phosphate 0.2 M, pH 7.4,
  ◦ 0.1 ml de solvant,
  ◦ 0.1 ml de S9-Mix.
  ◦ 0.1 ml de la préparation de l'élément à tester à la concentration la plus élevée,

- Une fraction de 2 ml d'agar top pour *S. typhimurium* est utilisée pour contrôler sa stérilité.
- Mélanger et verser sur la surface de la gélose de fond préalablement répartie dans des boîtes de Pétri.
- Incuber à 37 ° C $\pm$ 2 ° C pendant 48 à 72 heures.
- Le test est réalisé en triple.
- Aucune croissance bactérienne ne doit être observée.

Pour au moins 5 concentrations de l'extrait à tester, un test sans activation métabolique et un test avec activation métabolique ont été réalisés.

Expression des résultats et interprétation

De nombreux critères permettent de déterminer si un résultat est positif, notamment une augmentation du nombre de révertants corrélée à la dose d'item à tester, ou une augmentation reproductible du nombre de révertants à une ou plusieurs concentrations, avec ou sans activation métabolique.

- L'élément à tester est considéré comme mutagène si à l'issue des étapes de vérification, il a été obtenu, de manière reproductible, une relation dose-effet sur une ou certaines des 5 souches avec et / ou sans activation métabolique. La mutagénicité n'est prise en compte pour une concentration donnée que lorsque le nombre de révertants est au moins égal au double du taux de réversion spontanée pour les souches TA98, TA100 et TA102 (R > 2) et au triple du taux spontané de réversion pour les souches TA1535 et TA1537 (R ≥ 3).
- L'élément de test est considéré comme non mutagène si, à l'issue du test 1 et du test 2, le taux de révertants est toujours resté inférieur au double du taux de réversion spontanée pour toutes les concentrations de l'élément de test testé, pour les souches TA98, TA100 et TA102 (R <2) et inférieur au triple du taux de réversion spontanée pour les souches TA1535 et TA1537 (R <3), avec et sans activation métabolique, et à condition qu'il ait été vérifié que l'absence de l'effet mutagène n'est pas liée à la toxicité des concentrations testées.

L'étude préliminaire n'a montré aucune cytotoxicité de l'élément de test ; par conséquent, cette gamme de concentrations a été utilisée pour le test de génotoxicité 1.

En fonction du résultat obtenu pour le test 1, il a été décidé d'utiliser la même gamme de dilution pour le test 2. L'analyse des révertants montre que:

- Aucun effet cytotoxique n'a été observé,
- Aucune concentration de l'extrait à tester n'a montré un rapport R supérieur ou égal au moins au double du taux de réversion spontanée pour TA98, TA100 et TA102 et au triple du taux de réversion spontanée pour TA1535 et TA1537, avec et sans activation métabolique,
- Aucune réponse à la dose n'a été observée, quels que soient le système de test ou les conditions du test.

Au vu des résultats obtenus au cours de cette étude, l'hydrolysat peptidique selon l'exemple 1 peut être considéré comme ne présentant aucune activité mutagène ni pro-mutagène.

### 2. Test de photo-toxicité *in vitro* 3T3 NRU

Le principe du test a pour base la comparaison de la cytotoxicité de l'hydrolysat peptidique selon l'exemple 1 en présence et en absence d'une dose non cytotoxique d'UVA, sur des cellules en culture. La cytotoxicité est évaluée par la détermination de la viabilité cellulaire à l'aide d'un colorant vital : le rouge neutre, 24h après le traitement avec les éléments de référence et l'extrait de *M. peregrina* selon l'invention avec ou sans irradiation aux UVA. Les cellules utilisées sont des fibroblastes d'embryon de souris de lignée Balb/c 3T3 clone 31 (ATCC - CCL163). Le contrôle positif est une solution de chlorpromazine (Numéro CAS : 69-09-0). Le contrôle négatif est un diluant de l'extrait testé et de référence (solution saline tamponnée +/- 1% solvant). L'hydrolysat peptidique a été testé à 8 concentrations sur au moins quatre puits de culture par concentration étudiée, en présence ou en absence d'UVA. Les fibroblastes ont été trypsinés et deux plaques de culture 96 puits ont été ensemencées à raison de 100 μl d'une suspension cellulaire à $2.10^5$ cellules/ml (soit $2.10^6$ cellules par puits) dans du milieu de culture complet.

Les plaques ensemencées ont été incubées à l'étuve 24 heures à 37°C, 5% $CO_2$. A la fin de l'incubation, la semi-confluence du tapis cellulaire a été vérifiée. Les dilutions ont été préparées juste avant leur dépôt sur les cellules. Le PH de la plus forte concentration a été mesuré ; il est compris entre 6.5 et 7.8. Le milieu de culture a été éliminé, chaque puits a été préalablement rincé délicatement avec 150 μl de PBS maintenu à température ambiante avant d'être traité avec 100 μl de chaque dilution de l'extrait ou de référence. Les plaques de cultures ont été incubées à l'obscurité durant 1h ± 5 minutes à 37°C et 5 % de COz. L'irradiation a été réalisée à l'aide d'un irradiateur solaire BIO SUN (Vilber Lourmat RMX3W). Le BIO SUN est un système qui contrôle l'irradiation UV à l'aide d'un microprocesseur programmable. Le système suit en continu l'émission de lumière UV. L'irradiation s'arrête automatiquement quand l'énergie délivrée est égale à l'énergie programmée. L'irradiation spectrale du dispositif à l'essai a été mesurée dans la gamme de longueurs d'onde 250 à 700 nanomètres avec un spectroradiomètre calibré.

Une des 2 plaques a été irradiée avec son couvercle à température ambiante, et l'autre plaque a été protégée des UVA et conservée à température ambiante le temps de l'irradiation. Après l'irradiation, le milieu de traitement a été aspiré et les cellules ont été rincées. Puis 100 μl de milieu de culture complet ont été ajoutés doucement et les plaques ont été incubées pendant 18 à 22h à 37°C et 5 % de $CO_2$. Le lendemain la viabilité cellulaire (croissance, morphologie, intégrité de la monocouche) a été évaluée par observations sur un microscope à contraste de phase. Le milieu de culture a été éliminé, chaque puits a été préalablement rincé et maintenu à température ambiante avant d'être traité avec 100 μl de la solution de coloration. Les plaques ont été remises à l'incubateur durant 3h dans les mêmes conditions. La solution colorante a été éliminée et les cellules ont été rincées, puis la solution de rinçage a été enlevée et 150 μl de

solution de désorption ont été ajoutés dans chaque puits. Les plaques ont été agitées jusqu'à la solubilisation complète des cristaux. Les absorbances ont été mesurées à 450 nm.

Validation du test :

La sensibilité des cellules aux UVA est contrôlée tous les 10 passages environ, par évaluation de leur viabilité après exposition à des doses croissantes d'irradiation. Les cellules sont mises en culture à la densité utilisée dans l'essai. Elles sont irradiées le jour suivant à la dose de 2.5 et 9 J/cm$^2$ et la viabilité cellulaire est déterminée un jour plus tard à l'aide du test NRU. Les cellules répondent aux critères de qualité si leur viabilité après irradiation à 5 J/cm$^2$ UVA est supérieure ou égale à 80 % de la viabilité des témoins maintenus à l'obscurité ; à la dose la plus forte de 9 J/cm$^2$ UVA, la viabilité doit être au moins égal à 50% de celle des témoins maintenus à l'obscurité.

Résultats :

Le contrôle négatif a une absorbance supérieure ou égale à 0.4. La chlorpromazine, contrôle positif, présente une Cl$_{50}$ comprise entre 0.1 et 2 $\mu$g/ml en présence d'UVA et comprise entre 7 et 90 $\mu$g/ml en absence d'UVA. Ces résultats permettent de valider le test. La concentration de l'hydrolysat peptidique de tourteau de *peregrina* donnant 50% de mort cellulaire en présence ou en absence d'UVA ne peut être estimée. La mortalité n'a jamais atteint 50%. La concentration de l'hydrolysat peptidique de tourteau de peregrina donnant 50% de viabilité cellulaire en présence ou en absence d'UVA ne peut être estimée. La viabilité est toujours supérieure à 50%.

Conclusion : Dans les conditions expérimentales retenues l'hydrolysat peptidique de tourteau de *peregrina* peut être considéré comme non phototoxique.

3. Evaluation du potentiel irritant oculaire par l'étude de la cytotoxicité *in vitro* selon la méthode de relargage du rouge neutre sur lignée cellulaire SIRC

Cette étude in vitro a pour base l'évaluation de la cytotoxicité de l'hydrolysat peptidique de tourteau de *peregrina* par détermination de la concentration entrainant 50% de mortalité cellulaire (Cl$_{50}$) sur une monocouche cellulaire à l'aide de la technique de relargage du rouge neutre. Les cellules utilisées sont des fibroblastes de cornée de lapin SIRC (ATCC - CCL60) exemptes de mycoplasmes.

L'hydrolysat peptidique a été dilué dans du sérum physiologique à 25% et 50%. Les fibroblastes ont été trypsinés et deux plaques de culture 24 puits ont été ensemencées à raison de 1 ml d'une suspension cellulaire à 2.10$^5$ cellules/ml dans du milieu de culture complet. Les plaques ensemencées ont été incubées à l'étuve une nuit à 37°C et 5% CO$_2$. A la fin de l'incubation, la confluence du tapis cellulaire a été vérifiée. La solution de coloration a été préparée à 0.5 mg/ml dans du milieu de culture complet. Le milieu de culture a été éliminé ; 1 ml de la solution colorante a été déposée dans chaque puits. Les plaques ont été remises à l'incubateur à 37°C et 5% CO2 pendant 3h +/- 15 minutes. Après ce temps de contact, la solution colorante a été éliminée et remplacée par 1 ml de milieu de culture complet par puits. Les plaques ont été maintenues à température ambiante pendant au moins 30 minutes afin de stabiliser le système avant le contact avec l'extrait ou la référence. Chaque puits a été rincé avec 2 ml de PBS, maintenu à température ambiante, puis 500 $\mu$l de chaque dilution d'hydrolysat peptidique ou de référence ont été déposées au contact du tapis cellulaire. Le temps de contact a été de 60 secondes (30 secondes pour le contrôle positif). Le traitement a été réalisé puits par puits avec déclenchement du chronomètre au moment du dépôt de l'hydrolysat peptidique ou la référence. La plaque a été agitée manuellement pendant toute la durée du traitement. Après 55 secondes (ou 25 secondes pour le contrôle positif), la dilution a été aspirée. A 60 ou 30 secondes précises, 5 rinçages successifs ont été effectués (5 $\times$ 2 ml de PBS maintenus à température ambiante). Le surnageant a été aspiré après chaque rinçage et après le dernier rinçage les puits sont restés dépourvus de milieu en attendant la phase de révélation. Après traitement complet de la plaque de culture, 1 ml de la solution de désorption a été déposée dans chaque puits. La plaque est agitée pendant environ 15 minutes jusqu'à l'obtention d'une coloration homogène. Les solutions obtenues pour chaque puits de culture ont été prélevées et réparties dans 2 puits d'une plaque 96 puits, soit 150 $\mu$l/puits.

Résultats :

La concentration de l'hydrolysat peptidique donnant 50% de mort cellulaire a été évaluée à >50%. Le pourcentage de mort cellulaire à 50% d'hydrolysat peptidique a été évalué à 17%.

Conclusion : Dans les conditions expérimentales retenues, la cytotoxicité de l'hydrolysat peptidique de tourteau de *peregrina* peut être classée comme cytotoxicité négligeable.

4. Evaluation de la compatibilité cutanée d'un hydrolysat peptidique après application unique sous pansement occlusif pendant 48 heures sous contrôle dermatologique.

Le but de cette étude est d'évaluer le degré de compatibilité cutanée de l'hydrolysat peptidique par test épicutané, réalisé au niveau de la face antéro-externe du bras pendant 48 heures ; et d'une manière générale évaluer la capacité de l'hydrolysat peptidique à maintenir la peau en bon état. 10 volontaires sains, féminin ou masculin, âgés de 18 à 65 ans, n'ayant ni la peau sèche ni la peau sensible et indemnes de toute lésion dermatologique sur la zone de traitement devaient être inclus dans l'étude. La compatibilité cutanée de l'hydrolysat peptidique, préparé sous forme de lotion avec 5% de l'extrait *peregrina* selon l'exemple 1 et 95% d'un mélange Propanediol/Sorbitol, a été évaluée 48 heures après l'application initiale entre 30 à 40 minutes après le retrait du pansement. Le comptage des réactions cutanées (érythème et oedème) a été effectué de 0 à 3 selon les échelles suivantes :

[Tableau 21]

| Cotation | Erythème (E) | Œdème (Oe) |
|---|---|---|
| 0 | aucun érythème | aucun œdème |
| 0.5 | Érythème à peine perceptible, coloration très légèrement rosée sur une partie de la zone depatchage | œdème palpable à peine perceptible |
| 1 | Érythème léger, coloration rosée sur toute la surface de patchage | œdème palpable et visible |
| 2 | Érythème modéré, coloration nette sur toute la surface de patchage | œdème net avec ou sans papules ou vésicules |
| 3 | Érythème important, coloration intense sur toute la zone de patchage | œdème important diffusant en dehors de la zone de patchage avec ou sans papules ou vésicules |

Toute autre réaction cutanée (bulles, papules, vésicules, sécheresse, desquamation, rugosité, effet savon...) a été évaluée selon l'échelle suivante et rapportée de manière descriptive :

- 0 : aucune réaction,
- 0.5 : très léger
- 1 : léger
- 2 : modéré
- 3 : important.

A la fin de l'étude, un indice d'irritation moyen (I.I.M.) a été calculé selon la formule suivante :

[Math. 4]

$$\text{I.I.M.} = \text{Somme des réactions cutanées (E + Oe + bulles + papules + vésicules) / Nombre de volontaires analysés}$$

L'I.I.M. obtenu a permis de classer l'extrait testé selon le barème présenté dans le tableau ci-après :

$$\text{I.I.M.} \leq 0.20$$

Non irritant

$$0.20 < \text{I.I.M.} \leq 0.50$$

Légèrement irritant

$$0.50 < I.I.M. \leq 2$$

Moyennement irritant

$$2 < I.I.M. \leq 3$$

Très irritant

Résultats : L'indice d'irritation Moyen (I.I.M.) de l'hydrolysat peptidique de tourteau de *peregrina* est égal à : 0.

Conclusion : L'hydrolysat peptidique de tourteau de *peregrina* peut être considéré comme non irritant après 48 heures consécutives d'application chez 12 volontaires.

Conclusion générale des tests :

Les résultats des tests effectués ci-dessus sont concluants et démontrent pour l'hydrolysat peptidique selon l'exemple 1 :

    1) Les tests d'irritation oculaire et cutanée sont négatifs
    2) Les tests de phototoxicité sont négatifs
    3) Les tests de mutagénicité sont négatifs.

La sécurité de l'hydrolysat peptidique selon l'invention est démontrée et idéale pour une utilisation cosmétique topique à grande échelle sans restriction sur les populations cibles.

Exemple 22 : Activité de dépigmentation de la peau de l'extrait hydrolysé de tourteau

Le but du test est d'évaluer le potentiel dépigmentant et l'acceptabilité d'un produit cosmétique selon l'invention après 28 jours d'application. Le produit testé est la crème anti-rides décrite dans l'exemple 20.

Population analysée :
Nombre de volontaires inclus et analysés : 23

Genre féminin et âge moyen de 64 ans, compris entre 44 et 70 ans, ayant des lentigos au niveau du visage, du cou, du décolleté et/ou des mains.

Mode d'emploi retenu pour l'étude : 2 fois par jour, pendant 28 jours, appliquer une noisette de produit sur la peau propre du visage, du cou, du décolleté et des mains puis masser jusqu'à pénétration complète du produit ; éviter le contact avec les yeux ; appliquer le produit solaire fourni avant toute exposition au soleil.

Critères d'évaluation :

- Evaluation du potentiel dépigmentant : mesures mexamétriques au niveau de trois sites (zone pigmentée traitée, zone non pigmentée traitée, zone témoin non pigmentée non traitée), à J1 et J28.

- Remontée des sensations d'inconfort par les volontaires.

- Photographies à visée illustrative d'un lentigo au vidéodermoscope C-Cube (Pixience SAS, Toulouse, France) à J1 et J28 (transmises sous forme de planches contact par WeTransfer lors de la mise à disposition du rapport final).

- Acceptabilité cosmétique : questionnaire complété par le volontaire à J28.

Résultats de l'étude :

[Tableau 22]

| | Index mélanique (U.A.) | | | | | |
|---|---|---|---|---|---|---|
| | Zone pigmentée traitée | | Zone non pigmentée traitée | | Zone non pigmentée non traitée | |
| | J1 | J28 | J1 | J28 | J1 | J28 |
| Moyenne | 200.9 | 183.8 | 143.5 | 133. 8 | 122.9 | 121.4 |
| Ecart type | 42.8 | 35.3 | 27.2 | 22.7 | 33.4 | 32.7 |
| Médiane | 185.0 | 180.5 | 149.5 | 131. 0 | 116.0 | 117.5 |
| Minimum | 126.0 | 116.5 | 95.5 | 91.5 | 79.0 | 84.5 |
| Maximum | 289.5 | 251.5 | 197.0 | 173. 5 | 228.0 | 214.5 |
| % de variation | -8.5% | | -6.8% | | -1.2% | |
| Valeur de p* | 0.000 | | 0.001 | | 0.148 | |
| Significativité** | S | | S | | NS | |
| *Test de Wilcoxon pour données appariées :**S : Significatif (p $\leq$ 0.05) NS : Non Significatif (p > 0.05) | | | | | | |

L'analyse des mesures mexamétriques a permis de mettre en évidence une diminution statistiquement significative de l'index mélanique sur les 2 zones traitées (zone pigmentée et zone non pigmentée) à J28 par rapport à J1. Les variations enregistrées entre J28 et J1 au niveau de la zone non traitée ont été trouvées statistiquement non significatives.

Conclusion : Dans les conditions de l'étude, le produit testé a présenté une efficacité dépigmentante significative après 28 jours d'utilisation.

## Revendications

1. Extrait du tourteau des graines de *Moringa peregrina* comprenant majoritairement un hydrolysat peptidique qui comprend des dérivés d'acides aminés, des acides aminés, des peptides et glycopeptides dont le poids moléculaire est compris entre 100 Da et 6 000 Da, préférentiellement entre 1 500 Da et 5 000 Da, préférentiellement encore entre 3 000 et 4 500 Da, **caractérisé en ce qu'**il est obtenu des graines non décortiquées et à maturité du fruit de *Moringa peregrina* par protéolyse chimique à pH supérieur à 13 pendant une durée d'environ 2 heures à une température comprise entre 16 et 25°C.

2. Extrait selon la revendication 1, **caractérisé en ce qu'**il comprend également entre 0.3 % et 3 % de composés volatiles, dont 50% de ces composés soit entre 0.15% et 1.5% de l'extrait est constitué de composés nitrilés légers, avec principalement l'isobutyronitrile et le méthyl-butanenitrile ; dont 5 à 10% de ces composés soit entre 0.015 et 0.3% de l'extrait est constitué de dérivés d'isothiocyanates, avec principalement l'isothiocyanate d'isopropyle et l'isothiocyanate d'isobutyle ; dont 1 à 5% de ces composés soit entre 0.003 et 0.15 % est constitué d'huile essentielle, avec principalement de l'Eucalyptol, du Menthol et du Benzaldéhyde.

3. Procédé d'obtention d'un extrait de tourteau des graines de *Moringa peregrina* selon l'une des revendications 1 ou 2, **caractérisé en ce qu'**il comprend les étapes suivantes selon lesquelles :

a) on recueille les graines non décortiquées et récoltées à maturité du fruit de *Moringa peregrina* que l'on sèche pour obtenir un taux d'humidité interne inférieur à 8%,
b) on presse les graines séchées de sorte à séparer l'huile du reste de la graine de sorte à obtenir le tourteau comprenant moins de 6% en poids d'huile résiduelle,
c) on broie le tourteau obtenu à l'étape b),
d) on met en dispersion en phase aqueuse le tourteau broyé obtenu à l'étape c),
e) on réalise une protéolyse chimique de la dispersion aqueuse obtenue à l'étape d) pendant une durée d'environ 2 heures, à pH supérieur à 13 et à température comprise entre 16 et 25°C,
f) on neutralise la protéolyse pour stabiliser l'hydrolysat peptidique obtenu,
g) on récupère l'hydrolysat peptidique par séparation solide/liquide,

h) on purifie l'hydrolysat peptidique par ultrafiltration, puis éventuellement,
i) on effectue une lyophilisation de l'hydrolysat peptidique obtenu à l'étape h).

**4.** Procédé selon la revendication 3, **caractérisé en ce que** la séparation solide/liquide de l'étape g) est réalisée par différents procédés tels que centrifugation, essorage, filtration,

**5.** Procédé selon l'une des revendications 3 ou 4, **caractérisé en ce que** à l'étape h) l'ultrafiltration est effectuée avec un seuil de coupure compris entre 100 Da et 6000 Da et plus particulièrement entre 1500 Da et 5000 Da, et encore plus particulièrement entre 3000 Da et 4500 Da.

**6.** Composition cosmétique ou nutricosmétique, **caractérisée en ce qu'**elle comprend, à titre d'agent actif, une quantité efficace d'un extrait du tourteau des graines de *Moringa peregrina* selon l'une des revendications 1 ou 2, et un excipient physiologiquement acceptable.

**7.** Composition selon la revendication 6, **caractérisée en ce qu'**elle est une composition cosmétique formulée pour être appliquée topiquement sur la peau et **en ce que** l'extrait de tourteau des graines de *Moringa peregrina* est présent dans la composition à une concentration de 0.002 à 20% en poids par rapport au poids total de la composition, préférentiellement de 0.01 à 10%.

**8.** Composition selon la revendication 6, **caractérisée en ce qu'**elle est une composition nutricosmétique formulée pour être ingérée et **en ce que** l'extrait de tourteau des graines de *Moringa peregrina* est présent dans la composition à une concentration de 0.01 à 100% en poids par rapport au poids total de la composition.

**9.** Utilisation cosmétique ou nutricosmétique d'une composition selon l'une des revendications 6 à 8, pour améliorer l'aspect de la peau, des muqueuses ou des phanères, prévenir et/ou lutter contre la sécheresse de la peau et des muqueuses, prévenir et/ou à lutter contre les signes cutanés du vieillissement et/ou du photo-vieillissement, favoriser le blanchiment de la peau et favoriser l'amincissement.

**Patentansprüche**

**1.** Extrakt aus dem Presskuchen der Samen von *Moringa peregrina,* der überwiegend ein Peptidhydrolysat umfasst, das Derivate von Aminosäuren, Aminosäuren, Peptiden und Glycopeptiden umfasst, deren Molekulargewicht zwischen 100 Da und 6000 Da, bevorzugt zwischen 1500 Da und 5000 Da, weiter bevorzugt zwischen 3 000 und 4 500 Da, liegt, **dadurch gekennzeichnet, dass** es aus den ungeschälten Samen der reifen Frucht von *Moringa peregrina* durch chemische Proteolyse bei einem pH-Wert von über 13 für eine Dauer von etwa 2 Stunden bei einer Temperatur zwischen 16 und 25 °C erhalten ist.

**2.** Extrakt gemäß Anspruch 1, **dadurch gekennzeichnet, dass** dieser weiterhin zwischen 0,3 % und 3 % flüchtige Verbindungen, von denen 50 % dieser Verbindungen bzw. zwischen 0,15 % und 1,5 % des Extrakts aus leichten Nitrilverbindungen bestehen, hauptsächlich aus Isobutyronitril und Methylbutannitril; von denen 5 bis 10 % dieser Verbindungen bzw. zwischen 0,015 bis 0,3 % des Extrakts aus Isothiocyanat-Derivaten bestehen, hauptsächlich aus Isopropylisothiocyanat und Isobutylisothiocyanat; 1 bis 5 % dieser Verbindungen bzw. 0,003 bis 0,15 % aus ätherischem Öl bestehen, hauptsächlich Eucalyptol, Menthol und Benzaldehyd, umfasst.

**3.** Verfahren zur Gewinnung eines Extrakts aus dem Presskuchen der Samen von *Moringa peregrina* gemäß einem der Ansprüche 1 bis 2, **dadurch gekennzeichnet, dass** es die folgenden Schritte umfasst:

a) Sammeln und Trocknen von ungeschälten Samen der reif geernteten Frucht von *Moringa peregrina,* um einen inneren Feuchtigkeitsgehalt von weniger als 8 % zu erhalten;
b) Pressung der getrockneten Samen, sodass sich das Öl vom Rest der Samen trennt, um den Presskuchen zu erhalten, der weniger als 6 Gew.-% Restöl umfasst,
c) Mahlen des in Schritt b) erhaltenen Presskuchens,
d) Dispersion des den in Schritt c) erhaltenen gemahlenen Presskuchens in einer wässrigen Phase,
e) Durchführung einer chemischen Proteolyse der in Schritt d) erhaltenen wässrigen Dispersion während einer Dauer von etwa 2 Stunden bei einem pH-Wert von über 13 und einer Temperatur zwischen 16 und 25°C,
f) Neutralisierung der Proteolyse, um das erhaltene Peptidhydrolysat zu stabilisieren,
g) Gewinnung des Peptidhydrolysats durch Fest-Flüssig-Trennung,

h) Reinigung des Peptidhydrolysats durch Ultrafiltration, dann gegebenenfalls
i) Gefriertrocknung des in Schritt h) erhaltenen Peptidhydrolysats.

4. Verfahren gemäß Anspruch 3, **dadurch gekennzeichnet, dass** die Fest-Flüssig-Trennung in Schritt g) durch verschiedene Verfahren wie Zentrifugieren, Schleudern, Filtrieren durchgeführt wird.

5. Verfahren gemäß einem der Ansprüche 3 oder 4, **dadurch gekennzeichnet, dass** in Schritt h) die Ultrafiltration mit einer Trenngrenze zwischen 100 Da und 6000 Da und bevorzugt zwischen 1500 Da und 5000 Da und weiter bevorzugt zwischen 3000 Da und 4500 Da durchgeführt wird.

6. Kosmetische oder nutrikosmetische Zusammensetzung, **dadurch gekennzeichnet, dass** sie als Wirkstoff eine wirksame Menge eines Extrakts aus dem Presskuchen der Samen von *Moringa peregrina* gemäß einem der Ansprüche 1 bis 2 und einen physiologisch annehmbaren Hilfsstoff umfasst.

7. Zusammensetzung gemäß Anspruch 6, **dadurch gekennzeichnet, dass** sie eine kosmetische Zusammensetzung ist, die formuliert ist, um topisch auf die Haut aufgetragen zu werden, und dass der Extrakt aus dem Presskuchen der Samen von *Moringa peregrina* in der Zusammensetzung in einer Konzentration von 0,002 bis 20 Gew.-%, bezogen auf das Gesamtgewicht der Zusammensetzung, vorzugsweise von 0,01 bis 10 Gew.-%, vorliegt.

8. Zusammensetzung gemäß Anspruch 6, **dadurch gekennzeichnet, dass** sie eine nutrikosmetische Zusammensetzung ist, die formuliert ist, um eingenommen zu werden, und dass der Extrakt aus dem Presskuchen der Samen von *Moringa peregrina* in der Zusammensetzung in einer Konzentration von 0,01 bis 100 Gew.-%, bezogen auf das Gesamtgewicht der Zusammensetzung, vorliegt.

9. Kosmetische oder nutrikosmetische Verwendung einer Zusammensetzung gemäß einem der Ansprüche 6 bis 8, um das Aussehen der Haut, der Schleimhäute oder der Anhangsgebilde zu verbessern, die Haut zu entspannen, zu beruhigen und zu entstressen und den Hautalterungserscheinungen und/oder der Fotoalterung vorzubeugen und/oder zu bekämpfen und zur Vorbeugung von Altersflecken.

**Claims**

1. An extract of *Moringa peregrina* seed cake predominantly comprising a peptide hydrolyzate which comprises amino acid derivatives, amino acids, peptides and glycopeptides with a molecular weight of between 100 Da and 6000 Da, preferentially between 1500 Da and 5000 Da, more preferentially between 3000 Da and 4500 Da, and **characterized in that** it is obtained from the unshelled seeds, when the fruit of *Moringa peregrina* is ripe, by chemical proteolysis at a pH of greater than 13 for a time of about 2 hours at a temperature of between 16 and 25°C.

2. The extract as claimed in claim 1, **characterized in that** it also comprises between 0.3% and 3% of volatile compounds, of which 50% of these compounds, i.e. between 0.15% and 1.5% of the extract, is constituted of light nitrile compounds, mainly isobutyronitrile and methylbutanenitrile; of which 5% to 10% of these compounds, i.e. between 0.015% and 0.3% of the extract, is constituted of isothiocyanate derivatives, mainly isopropyl isothiocyanate and isobutyl isothiocyanate; of which 1% to 5% of these compounds, i.e. between 0.003% and 0.15%, is constituted of essential oil, mainly of eucalyptol, menthol and benzaldehyde.

3. A process for obtaining an extract of *Moringa peregrina* seed cake as claimed in either of claims 1 and 2, **characterized in that** it comprises the following steps in which:

a) the unshelled seeds, harvested when the fruit of *Moringa peregrina* is ripe, are collected and dried to obtain an internal moisture content of less than 8%,
b) the dried seeds are pressed so as to separate the oil from the rest of the seed, so as to obtain the cake comprising less than 6% by weight of residual oil,
c) the cake obtained in step b) is milled,
d) the milled cake obtained in step c) is dispersed in aqueous phase,
e) chemical proteolysis of the aqueous dispersion obtained in step d) is performed for a time of about 2 hours, at a pH of greater than 13 and at a temperature of between 16 and 25°C,
f) the proteolysis is neutralized to stabilize the peptide hydrolyzate obtained,
g) the peptide hydrolyzate is recovered by solid/liquid separation,

h) the peptide hydrolyzate is purified by ultrafiltration and then, optionally,
i) lyophilization of the peptide hydrolysate obtained in step h) is performed.

4. The process as claimed in claim 3, **characterized in that** the solid/liquid separation of step g) is performed by various processes such as centrifugation, dewatering or filtration.

5. The process as claimed in either of claims 3 and 4, **characterized in that** the ultrafiltration in step h) is performed with a cutoff threshold between 100 Da and 6000 Da, more particularly between 1500 Da and 5000 Da and even more particularly between 3000 Da and 4500 Da.

6. A cosmetic or nutricosmetic composition **characterized in that** it comprises, as active agent, an effective amount of an extract of *Moringa peregrina* seed cake as claimed in either of claims 1 and 2, and a physiologically acceptable excipient.

7. The composition as claimed in claim 6, **characterized in that** it is a cosmetic composition formulated for topical application to the skin and **in that** the extract of *Moringa peregrina* seed cake is present in the composition in a concentration of from 0.002% to 20% by weight, preferentially from 0.01 % to 10%, relative to the total weight of the composition.

8. The composition as claimed in claim 6, **characterized in that** it is a nutricosmetic composition formulated for ingestion and **in that** the extract of *Moringa peregrina* seed cake is present in the composition in a concentration of from 0.01 % to 100% by weight relative to the total weight of the composition.

9. The cosmetic or nutricosmetic use of a composition as claimed in one of claims 6 to 8, for improving the appearance of the skin, mucous membranes or the integuments, for preventing and/or combating dryness of the skin and the mucous membranes, for preventing and/or combating the signs of aging and/or photoaging of the skin, for promoting bleaching of the skin and for promoting slimming.

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 296879
- FR 2776519
- FR 2825267
- FR 3076460
- KR 20130088224
- FR 2946879
- US 7803750 B2, HALE L.

**Littérature non-brevet citée dans la description**

- **OLSON, M. E.** Combining Data from DNA Sequences and Morphology for a Phylogeny of Moringaceae (Brassicales). *Systematic Botany,* 2002, vol. 27 (1), 55-73
- **HASSANEIN, A. M. A. ; SOQEE, A. A. et al.** Morphological and genetic diversity of Moringa oleifera and Moringa peregrina génotypes. *Horticulture, Environment and Biotechnology,* 2018, vol. 59 (2), 251-261
- **ALAKLABI, A.** Genetic diversity of Moringa peregrina species in Saudi Arabia with ITS sequences. *Saudi Journal of Biological Sciences,* 2015, vol. 22, 186-190
- **GOMAA N. H.** Seed germination, seedling traits, and seed bank of the tree Moringa peregrina (Moringaceae) in a hyper-arid environment. *American Journal of Botany,* 2011, vol. 98 (6), 1024-1030
- **ABDEL-KADER, M. S. ; HAZAZI A. M. A. ; ELMAKKI O. A.** A survey on the traditional plants used in Al Kobah village. *Saudi Pharmaceutical Journal,* 2018, vol. 26 (6), 817-821
- **AQEEL A. A. M. ; TARIQ M. ; MOSSA J. S. ; AL-YAHYA M. A. ; AL-SAID M. S.** Plants used in Arabia Folk medicine. *Report submitted to Saudi Arabian National Cenre for Science and Technology,* 1984
- **GHAZANFAR S. A.** Handbook of Arabian Medicinal Plants. CRC Press, 1994
- **GHAZANFAR S.A.** Plants of Economic Importance. 1998, 247-251
- Végétation of the Arabian Peninsula. **GHAZANFAR, S.A.** Geobotany. Kluwer Academic Publishers, vol. 25, 241-264
- **GHAZANFAR S. A. ; RECHINGER B.** Two multi-purpose seed oils form Oman. Plants for Food and Medicine. *Paper presented at the joint meeting of the Society for Economic Botany and International Society for Ethnopharmacology,* Juillet 1996, vol. 1-7
- **AL-DABBAS M.** Antioxidant activity of différent extracts from the aerial part of Moringa peregrina (Forssk.) Fiori, from Jordan. *Pakistan Journal of Pharmaceutical Sciences,* 2017, vol. 30 (6), 2151-2157
- International Nomenclature of Cosmetic Ingrédients. L'INCI Dictionary & Handbook. The Personal Care Products Council Inc, 2010
- **KJAER et al.** Isothiocyanates in Myrosinase-treated seed extracts of Moringa peregrina. *Phytochemistry,* 1979, vol. 18, 1485-1487
- **AFSHARYPUOR et al.** Volatile Constituents of the Seed Kernel and Leaf of Moringa peregrina (Forssk.) Fiori, Agricolt. Cultivated in Chabahar (Iran). *Iranian Journal of Pharmaceutical Sciences,* 2010, vol. 6 (2), 141-144
- **DEHSHAHRI S. et al.** Détermination of volatile glucosinate dégradation products in seed coat, stem and in vitro cultures of Moringa peregrina (Forssk.) Fiori, ScienceOpen. *Research in Pharmaceutical Sciences,* 2012, vol. 7 (1), 51-56
- **ABD RANI N.Z. ; KHARAINA, H. ; KUMOLOSAS), E.** Moringa genus: A review of Phytochemistry and Pharmacology, Frontiers. *Pharmacology,* 2018, vol. 9
- **RONCOSO-ROJAS, R. ; TIZNADO-HERNANDEZ, M. E.** Natural compounds to control fungal diseases in fruits & vegetables. *TRONCOSO-ROJAS,* 2007
- Recent advances in alternative postharvest technologies to control fungal diseases in fruits & vegetables. Transworld Research Network, 127-156
- **TRONCOSO-ROJAS, R. et al.** Analysis of the isothiocyanates présent in cabbage leaves extract and their potential application to control Alternaria rot in bell peppers. *Food Research International,* 2005, vol. 38, 701-708

- **PARK, E.J. et al.** Inhibition of lipopolysaccharide induced cyclooxygenase-2 expression and inducible nitric oxyde synthase by 4-[(2-O-acetyl-$\alpha$-L-rhamnosyloxy)benzyl]isothiocyanate. *M. oleifera, Nutrition and Cancer,* 2011, vol. 63 (6), 971-982
- **RAJAN, T. S. et al.** Anticancer activity of glucomoringin isothiocyanate in human malignant astrocytoma cells. *Fitoterapa,* 2016, vol. 110, 1-7
- **PADLA E. P. et al.** Antimicrobial isothiocyanates from the seeds of Moringa oleifera Lam. *Zeitschrift für Naturforschung C,* 2012, vol. 67, 557-564
- **WATERMAN, C. et al.** Stable, water extractable isothiocyanates from Moringa oleifera leaves attenuate inflammation in vitro. *Phytochemistry,* 2014, vol. 103, 114-122
- **KADONO, S. et al.** The Rôle of the Epidermal Endothelin Cascade in the Hyperpigmentation Mechanism of Lentigo Senilis. *Journal of Investigative Dermatology,* 2001, vol. 116 (4), 571-577
- **KAWAHARA N. et al.** About the Component of Snow Tea, Collection of Pharmaceutical. *Society of Japan annual convention Subject matter,* 2005, 159
- **PANG, Y. ; GENG, J. ; OIN, Y. et al.** Endothelin-1 increases melanin synthesis in an established sheep skin mélanocyte culture. *Vitro Cellular & Developmental Biology - Animal,* 2016, vol. 52, 749-756
- **HACHIYA, AKIRA et al.** Biochemical Characterization of Endothelin-converting Enzyme-1$\alpha$ in Cultured Skin-derived Cells and Its Postulated Role in the Stimulation of Melanogenesis in Human Epidermis. *Journal of Biological Chemistry,* vol. 277 (7), 5395-540
- **HIRATA, Y. et al.** Cellular mechanism of action by a novel vasoconstrictor endothelin in cultured rat vascular smooth muscle cells. *Biochemical and Biophysical Research Communications,* 1988, vol. 154 (3), 868-875
- **SHALINKUMAR P. et al.** H2S Médiates the Vasodilator Effect of Endothelin-1 in the Cérébral Circulation. *American Journal of Physiology. Heart Circulatory Physiology,* 2008, vol. 315, 1759-1764
- **AHMADJAN M. et al.** Lipolysis in Adipocytes. *The International Journal of Biochemistry and Cell Biology,* 2010, vol. 42 (5), 555-559
- **HIRAI K. et al.** Biological Evaluation of a Lipid-Mobilizing Factor Isolated from the Urine of Cancer Patients. *Cancer Research,* 1998, vol. 58 (11), 2359-2365
- **GHADA F. M. et al.** Highlights in Pathogenesis of Vitiligo. *World Journal of Clinical Cases,* 2015, vol. 3 (3), 221-230
- **BRENNER M. et al.** The Protective Rôle of Melanin Against UV damage in human skin. *Photochemistry and Photobiology,* 2008, vol. 84 (3), 539-549
- **YAMAGUCHI Y. et al.** Mesenchymal-Epithelial Interactions in the Skin: Increased Expression of Dickkopf by Palmoplantar Fibroblasts Inhibits Melanocyte Growth and Différentiation. *Journal of Cell Biology,* 2004, vol. 165 (2), 275-285
- **SHAY, J. W.** Senescence an Immortalization: Role of Telomeres and Telomerase. *Carcinogenesis,* 2005, vol. 26 (5), 867-874
- **BLASCO M.** Telomere Length, Stem Cells and Aging. *Nature Chemical Biology,* 2007, vol. 3, 640-649
- *CHEMICAL ABSTRACTS,* 69-09-0